# EUROPEAN PATENT SPECIFICATION

(11) **EP 3 155 013 B1**
(45) Date of publication and mention of the grant of the patent: **27.03.2019**
(21) Application number: 15726647.9
(22) Date of filing: 10.06.2015
(51) Int. Cl.: C07K 16/24

(54) **METHOD FOR SELECTING ANTIBODIES WITH MODIFIED FCRN INTERACTION**
VERFAHREN ZUR AUSWAHL VON ANTIKÖRPERN MIT MODIFIZIERTER FCRN-INTERAKTION
PROCÉDÉ POUR SÉLECTIONNER DES ANTICORPS PRÉSENTANT UNE INTERACTION FCRN MODIFIÉE

(30) Priority: 12.06.2014 EP 14172180
(43) Date of publication of application: 19.04.2017
(73) Proprietor: F. Hoffmann-La Roche AG, 4070 Basel (CH)
(72) Inventor: FOGED JENSEN, Pernille, 2630 Taastrup (DK); KETTENBERGER, Hubert, 81369 München (DE); KOENIG, Maximiliane, 82335 Berg (DE); RAND, Kasper, 2000 Frederiksberg (DK); SCHLOTHAUER, Tilman, 82377 Penzberg (DE)
(74) Representative: Burger, Alexander
(86) International application number: PCT/EP2015/062899
(87) International publication number: WO 2015/189249

(56) References cited:
- EP-A1- 2 233 500
- US-A1- 2012 028 304
- N.N.: "Monoclonal antibody database", , 1 October 2007 (2007-10-01), pages 1-3, XP055144921, Retrieved from the Internet: URL:http://www.imgt.org/mAb-DB/query [retrieved on 2014-10-07]
- TILMAN SCHLOTHAUER ET AL: "Analytical FcRn affinity chromatography for functional characterization of monoclonal antibodies", MABS, vol. 5, no. 4, 1 July 2013 (2013-07-01), pages 576-586, XP055132983, ISSN: 1942-0862, DOI: 10.4161/mabs.24981
- "Abrilumab- IMGT/2D structure-DB card for INN9921 -", IMGT 2D structure database, 1 January 2013 (2013-01-01), pages 1-2, XP055144598, Retrieved from the Internet: URL:http://www.imgt.org/3Dstructure-DB/cgi /details.cgi?pdbcode=9921 [retrieved on 2014-10-06]

### SEQUENCE LISTING

<110> F. Hoffmann-La Roche AG
<120> Method for selecting antibodies with modified FcRn interaction
<130> P32178-WO
<150> EP14172180.3
   <151> 2014-06-12
<160> 14
<170> PatentIn version 3.5
<210> 1
   <211> 125
   <212> PRT
   <213> Artificial Sequence
<220>
   <223> humanized anti-digoxigenin antibody VH
<400> 1
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asp Tyr
               20                  25                  30
   Ala Met Ser Trp Ile Arg Gln Ala Pro Gly Lys Gly Leu Glu Trp Val
           35                  40                  45
   Ser Ser Ile Asn Ile Gly Ala Thr Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Pro Gly Ser Pro Tyr Glu Tyr Asp Lys Ala Tyr Tyr Ser Met
               100                 105                 110
   Ala Tyr Trp Gly Gln Gly Thr Thr Val Thr Val Ser Ser
           115                 120                 125
<210> 2
   <211> 330
   <212> PRT
   <213> Homo sapiens
<400> 2
<210> 3
   <211> 107
   <212> **PRT**
   <213> **Artificial** Sequence
<220>
   <223> humanized anti-digoxigenin antibody VL
<400> 3
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Lys Asn Tyr
               20                  25                  30
   Leu Asn Trp Tyr Gln Gln Lys Pro Gly Lys Ala Pro Lys Leu Leu Ile
           35                  40                  45
   Tyr Tyr Ser Ser Thr Leu Leu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Ile Thr Leu Pro Pro
                   85                  90                  95
   Thr Phe Gly Gly Gly Thr Lys Val Glu Ile Lys
               100                 105
<210> 4
   <211> 107
   <212> **PRT**
   <213> Homo sapiens
<400> 4
<210> 5
   <211> 115
   <212> PRT
   <213> Artificial Sequence
<220>
   <223> Briakinumab VH amino acid sequence
<400> 5
<210> 6
   <211> 112
   <212> PRT
   <213> Artificial Sequence
<220>
   <223> Briakinumab VL amino acid sequence
<400> 6
   Gln Ser Val Leu Thr Gln Pro Pro Ser Val Ser Gly Ala Pro Gly Gln
   1               5                   10                  15
   Arg Val Thr Ile Ser Cys Ser Gly Ser Arg Ser Asn Ile Gly Ser Asn
               20                  25                  30
   Thr Val Lys Trp Tyr Gln Gln Leu Pro Gly Thr Ala Pro Lys Leu Leu
           35                  40                  45
   Ile Tyr Tyr Asn Asp Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ala Glu Asp Glu Ala Asp Tyr Tyr Cys Gln Ser Tyr Asp Arg Tyr Thr
                   85                  90                  95
   His Pro Ala Leu Leu Phe Gly Thr Gly Thr Lys Val Thr Val Leu Gly
               100                 105                 110
<210> 7
   <211> 119
   <212> PRT
   <213> Artificial Sequence
<220>
   <223> Ustekinumab VH amino acid sequence
<400> 7
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Thr Tyr
               20                  25                  30
   Trp Leu Gly Trp Val Arg Gln Met Pro Gly Lys Gly Leu Asp Trp Ile
           35                  40                  45
   Gly Ile Met Ser Pro Val Asp Ser Asp Ile Arg Tyr Ser Pro Ser Phe
       50                  55                  60
   Gln Gly Gln Val Thr Met Ser Val Asp Lys Ser Ile Thr Thr Ala Tyr
   65                  70                  75                  80
   Leu Gln Trp Asn Ser Leu Lys Ala Ser Asp Thr Ala Met Tyr Tyr Cys
                   85                  90                  95
   Ala Arg Arg Arg Pro Gly Gln Gly Tyr Phe Asp Phe Trp Gly Gln Gly
               100                 105                 110
   Thr Leu Val Thr Val Ser Ser
           115
<210> 8
   <211> 107
   <212> PRT
   <213> Artificial Sequence
<220>
   <223> Ustekinumab VL amino acid sequence
<400> 8
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ile Tyr Pro Tyr
                   85                  90                  95
   Thr Phe Gly Gln Gly Thr Lys Leu Glu Ile Lys
                       100                 105
<210> 9
   <211> 274
   <212> PRT
   <213> Homo sapiens
<400> 9
   Ala Glu Ser His Leu Ser Leu Leu Tyr His Leu Thr Ala Val Ser Ser
   1               5                   10                  15
   Pro Ala Pro Gly Thr Pro Ala Phe Trp Val Ser Gly Trp Leu Gly Pro
               20                  25                  30
   Gln Gln Tyr Leu Ser Tyr Asn Ser Leu Arg Gly Glu Ala Glu Pro Cys
           35                  40                  45
   Gly Ala Trp Val Trp Glu Asn Gln Val Ser Trp Tyr Trp Glu Lys Glu
       50                  55                  60
   Thr Thr Asp Leu Arg Ile Lys Glu Lys Leu Phe Leu Glu Ala Phe Lys
   65                  70                  75                  80
   Ala Leu Gly Gly Lys Gly Pro Tyr Thr Leu Gln Gly Leu Leu Gly Cys
                   85                  90                  95
   Glu Leu Gly Pro Asp Asn Thr Ser Val Pro Thr Ala Lys Phe Ala Leu
               100                 105                 110
   Asn Gly Glu Glu Phe Met Asn Phe Asp Leu Lys Gln Gly Thr Trp Gly
           115                 120                 125
   Gly Asp Trp Pro Glu Ala Leu Ala Ile Ser Gln Arg Trp Gln Gln Gln
       130                 135                 140
   Asp Lys Ala Ala Asn Lys Glu Leu Thr Phe Leu Leu Phe Ser Cys Pro
   145                 150                 155                 160
   His Arg Leu Arg Glu His Leu Glu Arg Gly Arg Gly Asn Leu Glu Trp
                   165                 170                 175
   Lys Glu Pro Pro Ser Met Arg Leu Lys Ala Arg Pro Ser Ser Pro Gly
               180                 185                 190
   Phe Ser Val Leu Thr Cys Ser Ala Phe Ser Phe Tyr Pro Pro Glu Leu
           195                 200                 205
   Gln Leu Arg Phe Leu Arg Asn Gly Leu Ala Ala Gly Thr Gly Gln Gly
   210 215 220
   Asp Phe Gly Pro Asn Ser Asp Gly Ser Phe His Ala Ser Ser Ser Leu
   225 230 235 240
   Thr Val Lys Ser Gly Asp Glu His His Tyr Cys Cys Ile Val Gln His
   245 250 255
   Ala Gly Leu Ala Gln Pro Leu Arg Val Glu Leu Glu Ser Pro Ala Lys
   260 265 270
   Ser Ser
<210> 10
   <211> 99
   <212> PRT
   <213> Homo sapiens
<400> 10
<210> 11
   <211> 38
   <212> PRT
   <213> Homo sapiens
<400> 11
   Gly Gly Thr Ala Ala Leu Gly Cys Leu Val Lys Asp Tyr Phe Pro Glu
   1 5 10 15
   Pro Val Thr Val Ser Trp Asn Ser Gly Ala Leu Thr Ser Gly Val His
               20                  25                  30
   Thr Phe Pro Ala Val Leu
           35
<210> 12
   <211> 14
   <212> PRT
   <213> Homo sapiens
<400> 12
   Gly Ala Leu Thr Ser Gly Val His Thr Phe Pro Ala Val Leu
   1               5                   10
<210> 13
   <211> 18
   <212> PRT
   <213> Homo sapiens
<400> 13
   Tyr Ser Leu Ser Ser Val Val Thr Val Pro Ser Ser Ser Leu Gly Thr
   1               5                   10                  15
   Gln Thr
<210> 14
   <211> 16
   <212> PRT
   <213> Homo sapiens
<400> 14
   Leu Ser Ser Val Val Thr Val Pro Ser Ser Ser Leu Gly Thr Gln Thr
   1               5                   10                  15

## Description

The current application is in the field of antibody-FcRn interaction engineering. Herein is reported a method for the generation and selection of antibodies with engineered antibody FcRn interaction.

### Background of the Invention

The neonatal Fc-receptor (FcRn) is important for the metabolic fate of antibodies of the IgG class in vivo. The FcRn functions to salvage IgG from the lysosomal degradation pathway, resulting in reduced clearance and increased half-life. It is a heterodimeric protein consisting of two polypeptides: a 50 kDa class I major histocompatibility complex-like protein (a-FcRn) and a 15 kDa β2-microglobulin (β2m). FcRn binds with high affinity to the CH2-CH3 portion of the Fc-region of an antibody of the class IgG. The interaction between an antibody of the class IgG and the FcRn is pH dependent and occurs in a 1:2 stoichiometry, i.e. one IgG antibody molecule can interact with two FcRn molecules via its two heavy chain Fc-region polypeptides (see e.g. Huber, A.H., et al., J. Mol. Biol. 230 (1993) 1077-1083).

Houde, D., et al., reported the characterization of IgG1 conformation and conformational dynamics by hydrogen/deuterium exchange mass spectrometry (Anal. Chem. 81 (2009) 2644-2651). The importance of neonatal FcR in regulating the serum half-life of therapeutic proteins containing the Fc domain of human IgG1: a comparative study of the affinity of monoclonal antibodies and Fc-fusion proteins to human neonatal FcR was reported by Suzuki, T., et al. (J. Immunol. 184 (2010) 1968-1976). Wang, W., et al. reported that monoclonal antibodies with identical Fc sequences can bind to FcRn differentially with pharmacokinetic consequences (Drug Metabol. Dispos. 39 (2011) 1469-1477).

An analytical FcRn affinity chromatography for functional characterization of monoclonal antibodies was reported by Schlothauer, T., et al. (mAbs 5 (2013) 576-586).

Houde, D., et al. reported that post-translational modifications differentially affect IgG1 conformation and receptor binding (Mol. Cell Proteom. 9 (2010) 1716-1728).

In EP 14 165 987.0 the in vitro prediction of in vivo half-life is reported.

In EP 2 233 500 optimized Fc variants are reported. Antibodies with modified isoelectric points are reported in US 2012/028304.

### Summary of the Invention

It has been found that the regions including residues 1-23, 145-174, 180-197 in the heavy chain as well as residues 55-83 in the light chain (Kabat (variable domain) and EU index (constant region) numbering, respectively) of a monoclonal full length antibody take part in the interaction of the full length antibody with the human neonatal Fc receptor (FcRn).

Thus, the invention is a method for selecting a full length antibody comprising the following steps:
a) generating from a parent full length antibody a plurality of variant full length antibodies by randomizing one or more amino acid residues selected from the group of amino acid residues consisting of the residues at positions 1-23 in the heavy chain variable domain (numbering according to Kabat), the residues at positions 55-83 in the light chain variable domain (numbering according to Kabat), the residues at positions 145-174 in the first heavy chain constant domain (numbering according to EU index) and the residues at positions 180-197 in the first heavy chain constant domain (numbering according to EU index),
b) determining the binding strength of each of the full length antibodies from the plurality of antibodies to the human neonatal Fc receptor (FcRn), and
c) selecting a full length antibody from the plurality of variant full length antibodies that has a different binding strength to the FcRn than the parent full length antibody.

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat), at positions 161-174 in the first heavy chain constant domain (numbering according to EU index) and at positions 181-196 in the first heavy chain constant domain (numbering according to EU index).

In one embodiment the one or more amino acid residues are selected from the amino acid residues at positions 1-23 in the heavy chain variable domain (numbering according to Kabat).

In one embodiment the one or more amino acid residues are selected from the amino acid residues at positions 3-18 in the heavy chain variable domain (numbering according to Kabat).

In one embodiment the one or more amino acid residues are selected from the amino acid residues at positions 5-18 in the heavy chain variable domain (numbering according to Kabat).

In one embodiment the one or more amino acid residues are selected from the amino acid residues at positions 145-174 in the first heavy chain constant domain (numbering according to EU index).

In one embodiment the one or more amino acid residues are selected from the amino acid residues at positions 161-174 in the first heavy chain constant domain (numbering according to EU index).

In one embodiment the one or more amino acid residues are selected from the amino acid residues at positions 180-197 in the first heavy chain constant domain (numbering according to EU index).

In one embodiment the one or more amino acid residues are selected from the amino acid residues at positions 181-196 in the first heavy chain constant domain (numbering according to EU index).

In one embodiment the one or more amino acid residues are selected from the amino acid residues at positions 182-197 in the first heavy chain constant domain (numbering according to EU index).

In one embodiment the one or more amino acid residues are selected from the amino acid residues at positions 55-83 in the light chain variable domain (numbering according to Kabat).

In one embodiment the one or more amino acid residues are selected from the amino acid residues at positions 55-73 in the light chain variable domain (numbering according to Kabat).

In one embodiment the one or more amino acid residues are selected from the amino acid residues at positions 57-71 in the light chain variable domain (numbering according to Kabat).

In one embodiment the amino acid residues are selected from the group of amino acid residues comprising amino acid residues 6, 162, 164, 165, 191, 194, 195 and 196 in the heavy chain and amino acid residues 57 and 60 in the light chain (Kabat (variable domain) and EU index (constant region) numbering, respectively).

In one embodiment the amino acid residue at position 6 of the heavy chain variable domain is randomized to Q.

In one embodiment the amino acid residue at one or more of the amino acid positions 162, 164, 165, 191, 194, 195 and 196 of the heavy chain first constant domain (CHI) is randomized independently of each other to an acidic amino acid residue.

In one embodiment the amino acid residue at one or more of the amino acid positions 57 and 60 of the light chain variable domain is randomized independently of each other to a basic amino acid residue.

In one embodiment of all aspects as reported herein the determining the binding strength of each of the full length antibodies from the plurality of antibodies to the human neonatal Fc receptor (FcRn) is in the absence of the respective antigen of the antibodies.

In one embodiment of all aspects as reported herein the determining the binding strength of each of the full length antibodies from the plurality of antibodies to the human neonatal Fc receptor (FcRn) is done for the free, i.e. not-antigen complexed, antibody.

In one embodiment the antibody is a full length IgG antibody. In one embodiment the antibody is a full length IgG1 antibody.

A full length antibody comprises four antibody chains, two light chains (first light chain and second light chain) and two heavy chains (first heavy chain and second heavy chain). The first and second heavy chain as well as independently thereof the first and second light chain can be either identical or different with respect to their amino acid sequence.

In one embodiment the antibody is a one-armed antibody.

A one-armed antibody comprises i) one full length light chain, ii) one full length heavy chain that is paired with the full length light chain, and iii) one shortened heavy chain comprising at least a part of a hinge region, a CH2 domain and a CH3 domain that is paired with the full length heavy chain.

In one embodiment the antibody is a bispecific antibody.

In one embodiment one or more of the following amino acid positions are changed (numbering according to Kabat variable domain numbering and Kabat EU index numbering scheme, respectively) by replacing a charged amino acid residue with a neutral amino acid residue or by replacing a neutral amino acid residue with a charged amino acid residue independently of each other: heavy chain position 6, 16, 19, 57, 66, 83, 162, 164, 165, 191, 194, 195, 196, light chain position 57, 60.

In one embodiment one or more of the following amino acid positions are changed (numbering according to Kabat variable domain numbering and Kabat EU index numbering scheme, respectively) by replacing a charged amino acid residue with a neutral amino acid residue or by replacing a neutral amino acid residue with a charged amino acid residue independently of each other: heavy chain position 6, 16, 19, 162, 164, 165, 191, 194, 195, 196, light chain position 57, 60.

In one embodiment one or more of the following amino acid mutations (randomizations) are introduced (numbering according to Kabat variable domain numbering and Kabat EU index numbering scheme, respectively) independently of each other
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

In one embodiment the following amino acid mutation is introduced (numbering according to Kabat variable domain numbering and Kabat EU index numbering scheme, respectively)

### i) heavy chain E6Q.

In one embodiment the following amino acid mutation is introduced (numbering according to Kabat variable domain numbering and Kabat EU index numbering scheme, respectively)

### i) heavy chain T164E.

In one embodiment the following amino acid mutations are introduced (numbering according to Kabat variable domain numbering and Kabat EU index numbering scheme, respectively)

### i) heavy chain A162D and S165D.

In one embodiment the following amino acid mutation is introduced (numbering according to Kabat variable domain numbering and Kabat EU index numbering scheme, respectively) i) heavy chain G194E.

In one embodiment the following amino acid mutations are introduced (numbering according to Kabat variable domain numbering and Kabat EU index numbering scheme, respectively)

### i) heavy chain S191D and Q196D.

In one embodiment the following amino acid mutation is introduced (numbering according to Kabat variable domain numbering and Kabat EU index numbering scheme, respectively)

### i) light chain G57R.

In one embodiment the following amino acid mutations are introduced (numbering according to Kabat variable domain numbering and Kabat EU index numbering scheme, respectively)

### i) light chain G57K and S60K.

In one embodiment the following amino acid mutations are introduced (numbering according to Kabat variable domain numbering and Kabat EU index numbering scheme, respectively)
i) heavy chain T164E, and
ii) heavy chain A162D and S165D, and
iii) heavy chain G194E, and
iv) heavy chain S191D and Q196D.

In one embodiment the following amino acid mutations are introduced (numbering according to Kabat variable domain numbering and Kabat EU index numbering scheme, respectively)
i) heavy chain E6Q, and
ii) heavy chain T164E, and
iii) heavy chain A162D and S165D, and
iv) heavy chain G194E, S191D and Q196D, and
v) light chain G57K and S60K.

In one embodiment the following amino acid mutations are introduced (numbering according to Kabat variable domain numbering and Kabat EU index numbering scheme, respectively)
i) heavy chain E6Q, and/or
ii) heavy chain T164E, and/or
iii) heavy chain A162D and S165D, and/or
iv) heavy chain G194E, and/or
v) heavy chain T164E, A162D and S165D, and/or
vi) heavy chain G194E, S191D and Q196D, and/or
vii) light chain G57R, and/or
viii) light chain G57K and S60K.

In one embodiment the following amino acid mutations are introduced (numbering according to Kabat variable domain numbering and Kabat EU index numbering scheme, respectively)
i) E6Q in the first and/or second heavy chain, and/or
ii) a) T164E in the first heavy chain, and b) A162D and S165D in the second heavy chain, and/or
iii) a) G194E in the first heavy chain, and b) S191D and Q196D in the second heavy chain, and/or
iv) a) T164E, A162D and S165D in the first heavy chain, and b) G194Q, S191D and Q196D in the second heavy chain, and/or
v) a) E6Q in the first and/or second heavy chain, b) T164E, A162D and S165D in the first heavy chain, c) G194E, S191D and Q196D in the second heavy chain, and d) G57K and S60K in the first and/or second light chain.

In one embodiment the randomizing is by mutating the amino acid residue to a different amino acid residue from the same group of amino acid residues.

In one embodiment the randomizing is by mutating the amino acid residue to a different amino acid residue from a different group of amino acid residues.

In one embodiment one to fifteen amino acid residues are randomized. In one embodiment one to ten amino acid residues are randomized. In one embodiment one to five amino acid residues are randomized.

In one embodiment a full length antibody is selected from the plurality of full length antibodies that has an increased binding strength to the FcRn.

In one embodiment a full length antibody is selected from the plurality of full length antibodies that has a reduced binding strength to the FcRn.

In one embodiment the binding strength is the KD value.

In one embodiment the binding strength is the retention time on an FcRn affinity chromatography column with positive linear pH gradient elution.

In one embodiment the binding strength is the in vivo half-life.

One aspect as reported herein is a plurality of variant full length antibodies generated from a single parent full length antibody by randomizing one or more amino acid residues selected from the group of amino acid residues comprising positions 1-23 in the heavy chain variable domain (numbering according to Kabat), positions 55-83 in the light chain variable domain (numbering according to Kabat), positions 145-174 in the first heavy chain constant domain (numbering according to EU index) and positions 180-197 in the first heavy chain constant domain (numbering according to EU index).

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat), at positions 161-174 in the first heavy chain constant domain (numbering according to EU index) and at positions 181-196 in the first heavy chain constant domain (numbering according to EU index).

In one embodiment the one or more amino acid residues are selected from the amino acid residues at positions 1-23 in the heavy chain variable domain (numbering according to Kabat).

In one embodiment the one or more amino acid residues are selected from the amino acid residues at positions 3-18 in the heavy chain variable domain (numbering according to Kabat).

In one embodiment the one or more amino acid residues are selected from the amino acid residues at positions 5-18 in the heavy chain variable domain (numbering according to Kabat).

In one embodiment the one or more amino acid residues are selected from the amino acid residues at positions 145-174 in the first heavy chain constant domain (numbering according to EU index).

In one embodiment the one or more amino acid residues are selected from the amino acid residues at positions 161-174 in the first heavy chain constant domain (numbering according to EU index).

In one embodiment the one or more amino acid residues are selected from the amino acid residues at positions 180-197 in the first heavy chain constant domain (numbering according to EU index).

In one embodiment the one or more amino acid residues are selected from the amino acid residues at positions 181-196 in the first heavy chain constant domain (numbering according to EU index).

In one embodiment the one or more amino acid residues are selected from the amino acid residues at positions 182-197 in the first heavy chain constant domain (numbering according to EU index).

In one embodiment the one or more amino acid residues are selected from the amino acid residues at positions 55-83 in the light chain variable domain (numbering according to Kabat).

In one embodiment the one or more amino acid residues are selected from the amino acid residues at positions 55-73 in the light chain variable domain (numbering according to Kabat).

In one embodiment the one or more amino acid residues are selected from the amino acid residues at positions 57-71 in the light chain variable domain (numbering according to Kabat).

In one embodiment the amino acid residues are selected from the group of amino acid residues comprising amino acid residues 6, 162, 164, 165, 191, 194, 195 and 196 in the heavy chain and amino acid residues 57 and 60 in the light chain (Kabat (variable domain) and EU index (constant region) numbering, respectively).

In one embodiment the amino acid residue at position 6 of the heavy chain variable domain is randomized to Q.

In one embodiment the amino acid residue at one or more of the amino acid positions 162, 164, 165, 191, 194, 195 and 196 of the heavy chain first constant domain (CHI) is randomized to an acidic amino acid residue.

In one embodiment the amino acid residue at one or more of the amino acid positions 57 and 60 of the light chain variable domain is randomized to a basic amino acid residue.

In one embodiment one or more of the following amino acid randomizations are introduced independently of each other
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

In one embodiment the antibody is a full length IgG antibody. In one embodiment the antibody is a full length IgG1 antibody.

In one embodiment the randomizing is by mutating the amino acid residue to a different amino acid residue from the same group of amino acid residues.

In one embodiment the randomizing is by mutating the amino acid residue to a different amino acid residue from a different group of amino acid residues.

In one embodiment one to fifteen amino acid residues are randomized. In one embodiment one to ten amino acid residues are randomized. In one embodiment one to five amino acid residues are randomized.

In one embodiment a full length antibody is selected from the plurality of full length antibodies that has an increased binding strength to the FcRn.

In one embodiment a full length antibody is selected from the plurality of full length antibodies that has a reduced binding strength to the FcRn.

In one embodiment the binding strength is the KD value.

In one embodiment the binding strength is the retention time on an FcRn affinity chromatography column with positive linear pH gradient elution.

Another aspect as reported herein is the use of one or more amino acid mutations at positions selected from the group of positions comprising positions 1-23 in the heavy chain variable domain (numbering according to Kabat), positions 55-83 in the light chain variable domain (numbering according to Kabat), positions 145-174 in the first heavy chain constant domain (numbering according to EU index) and positions 180-197 in the first heavy chain constant domain (numbering according to EU index) for changing the in vivo half-life of a full length antibody.

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat), at positions 161-174 in the first heavy chain constant domain (numbering according to EU index) and at positions 181-196 in the first heavy chain constant domain (numbering according to EU index).

In one embodiment the one or more amino acid residues are selected from the amino acid residues at positions 1-23 in the heavy chain variable domain (numbering according to Kabat).

In one embodiment the one or more amino acid residues are selected from the amino acid residues at positions 3-18 in the heavy chain variable domain (numbering according to Kabat).

In one embodiment the one or more amino acid residues are selected from the amino acid residues at positions 5-18 in the heavy chain variable domain (numbering according to Kabat).

In one embodiment the one or more amino acid residues are selected from the amino acid residues at positions 145-174 in the first heavy chain constant domain (numbering according to EU index).

In one embodiment the one or more amino acid residues are selected from the amino acid residues at positions 161-174 in the first heavy chain constant domain (numbering according to EU index).

In one embodiment the one or more amino acid residues are selected from the amino acid residues at positions 180-197 in the first heavy chain constant domain (numbering according to EU index).

In one embodiment the one or more amino acid residues are selected from the amino acid residues at positions 181-196 in the first heavy chain constant domain (numbering according to EU index).

In one embodiment the one or more amino acid residues are selected from the amino acid residues at positions 182-197 in the first heavy chain constant domain (numbering according to EU index).

In one embodiment the one or more amino acid residues are selected from the amino acid residues at positions 55-83 in the light chain variable domain (numbering according to Kabat).

In one embodiment the one or more amino acid residues are selected from the amino acid residues at positions 55-73 in the light chain variable domain (numbering according to Kabat).

In one embodiment the one or more amino acid residues are selected from the amino acid residues at positions 57-71 in the light chain variable domain (numbering according to Kabat).

In one embodiment the amino acid residues are selected from the group of amino acid residues comprising amino acid residues 6, 162, 164, 165, 191, 194, 195 and 196 in the heavy chain and amino acid residues 57 and 60 in the light chain (Kabat (variable domain) and EU index (constant region) numbering, respectively).

In one embodiment the amino acid residue at position 6 of the heavy chain variable domain is mutated to Q.

In one embodiment the amino acid residue at one or more of the amino acid positions 162, 164, 165, 191, 194, 195 and 196 of the heavy chain first constant domain (CHI) is mutated independently of each other to an acidic amino acid residue.

In one embodiment the amino acid residue at one or more of the amino acid positions 57 and 60 of the light chain variable domain is mutated independently of each other to a basic amino acid residue.

In one embodiment one or more of the following amino acid mutations are introduced independently of each other
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

In one embodiment the antibody is a full length IgG antibody. In one embodiment the antibody is a full length IgG1 antibody.

In one embodiment the randomizing is by mutating the amino acid residue to a different amino acid residue from the same group of amino acid residues.

In one embodiment the randomizing is by mutating the amino acid residue to a different amino acid residue from a different group of amino acid residues.

In one embodiment one to fifteen amino acid residues are randomized. In one embodiment one to ten amino acid residues are randomized. In one embodiment one to five amino acid residues are randomized.

In one embodiment a full length antibody is selected from the plurality of full length antibodies that has an increased binding strength to the FcRn.

In one embodiment a full length antibody is selected from the plurality of full length antibodies that has a reduced binding strength to the FcRn.

In one embodiment the binding strength is the KD value.

In one embodiment the binding strength is the retention time on an FcRn affinity chromatography column with positive linear pH gradient elution.

A further aspect as reported herein is a variant full length antibody comprising two light chain polypeptides and two heavy chain polypeptides, wherein the variant antibody is derived from a parent full length antibody by introducing amino acid mutations at one or more positions selected from the group of positions comprising positions 1-23 in the heavy chain variable domain (numbering according to Kabat), positions 55-83 in the light chain variable domain (numbering according to Kabat), positions 145-174 in the first heavy chain constant domain (numbering according to EU index) and positions 180-197 in the first heavy chain constant domain (numbering according to EU index), and wherein the variant antibody has a different affinity for the human neonatal Fc receptor than the parent full length antibody.

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat), at positions 161-174 in the first heavy chain constant domain (numbering according to EU index) and at positions 181-196 in the first heavy chain constant domain (numbering according to EU index).

In one embodiment the one or more amino acid residues are selected from the amino acid residues at positions 1-23 in the heavy chain variable domain (numbering according to Kabat).

In one embodiment the one or more amino acid residues are selected from the amino acid residues at positions 3-18 in the heavy chain variable domain (numbering according to Kabat).

In one embodiment the one or more amino acid residues are selected from the amino acid residues at positions 5-18 in the heavy chain variable domain (numbering according to Kabat).

In one embodiment the one or more amino acid residues are selected from the amino acid residues at positions 145-174 in the first heavy chain constant domain (numbering according to EU index).

In one embodiment the one or more amino acid residues are selected from the amino acid residues at positions 161-174 in the first heavy chain constant domain (numbering according to EU index).

In one embodiment the one or more amino acid residues are selected from the amino acid residues at positions 180-197 in the first heavy chain constant domain (numbering according to EU index).

In one embodiment the one or more amino acid residues are selected from the amino acid residues at positions 181-196 in the first heavy chain constant domain (numbering according to EU index).

In one embodiment the one or more amino acid residues are selected from the amino acid residues at positions 182-197 in the first heavy chain constant domain (numbering according to EU index).

In one embodiment the one or more amino acid residues are selected from the amino acid residues at positions 55-83 in the light chain variable domain (numbering according to Kabat).

In one embodiment the one or more amino acid residues are selected from the amino acid residues at positions 55-73 in the light chain variable domain (numbering according to Kabat).

In one embodiment the one or more amino acid residues are selected from the amino acid residues at positions 57-71 in the light chain variable domain (numbering according to Kabat).

In one embodiment the amino acid residues are selected from the group of amino acid residues comprising amino acid residues 6, 162, 164, 165, 191, 194, 195 and 196 in the heavy chain and amino acid residues 57 and 60 in the light chain (Kabat (variable domain) and EU index (constant region) numbering, respectively).

In one embodiment the amino acid residue at position 6 of the heavy chain variable domain is mutated to Q.

In one embodiment the amino acid residue at one or more of the amino acid positions 162, 164, 165, 191, 194, 195 and 196 of the heavy chain first constant domain (CHI) is mutated independently of each other to an acidic amino acid residue.

In one embodiment the amino acid residue at one or more of the amino acid positions 57 and 60 of the light chain variable domain is mutated independently of each other to a basic amino acid residue.

In one embodiment one or more of the following amino acid mutations are introduced independently of each other
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

In one embodiment the antibody is a full length IgG antibody. In one embodiment the antibody is a full length IgG1 antibody.

In one embodiment the randomizing is by mutating the amino acid residue to a different amino acid residue from the same group of amino acid residues.

In one embodiment the randomizing is by mutating the amino acid residue to a different amino acid residue from a different group of amino acid residues.

In one embodiment one to fifteen amino acid residues are randomized. In one embodiment one to ten amino acid residues are randomized. In one embodiment one to five amino acid residues are randomized.

In one embodiment a full length antibody is selected from the plurality of full length antibodies that has an increased binding strength to the FcRn.

In one embodiment a full length antibody is selected from the plurality of full length antibodies that has a reduced binding strength to the FcRn.

In one embodiment the binding strength is the KD value.

In one embodiment the binding strength is the retention time on an FcRn affinity chromatography column with positive linear pH gradient elution.

In one embodiment the binding strength is the in vivo half-life.

One aspect as reported herein is a variant antibody that has a mutation at one or more of the following amino acid residues relative to its parent antibody
- residues 6, 162, 164, 165, 191, 194, 195 and 196 in the heavy chain
- residues 57 and 60 in the light chain
(numbering according to Kabat (variable domain) and EU index (constant region), respectively).

In one embodiment the variant antibody that has a mutation at one or more of the following amino acid residues relative to its parent antibody
- residues 162, 164, 165, 191, 194, 195 and 196 in the heavy chain
- residues 57 and 60 in the light chain
(numbering according to Kabat (variable domain) and EU index (constant region), respectively).

In one embodiment the variant antibody that has a mutation at one or more of the following amino acid residues relative to its parent antibody
- residues 162, 164, 165, 191, 194, 195 and 196 in the heavy chain
(numbering according to Kabat (variable domain) and EU index (constant region), respectively).

In one embodiment the antibody is a full length IgG antibody. In one embodiment the antibody is a full length IgG1 antibody.

In one embodiment the antibody is a bispecific antibody.

In one embodiment the antibody has the amino acid mutation E6Q in the heavy chain variable domain.

In one embodiment the antibody has an acidic amino acid at one or more of the amino acid positions 162, 164, 165, 191, 194, 195 and 196 of the heavy chain first constant domain (CHI). In one embodiment the acidic amino acid is D or E. In on embodiment the antibody has an acidic amino acid residue at two or more of the amino acid positions 162, 164, 165, 191, 194, 195 and 196 of the heavy chain first constant domain (CHI) whereby the acidic amino acid residues are selected independently of each other.

In one embodiment the antibody has a basic amino acid residue at one or both of the amino acid positions 57 and 60 of the light chain variable domain. In one embodiment the basic amino acid residue is K or R. In one embodiment the antibody has a basic amino acid residue at both of the positions 57 and 60 of the light chain variable domain whereby the basic amino acid residues are selected independently of each other.

In one embodiment the antibody has one or more of the following amino acid mutations independently of each other
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

### Detailed Description of the Invention

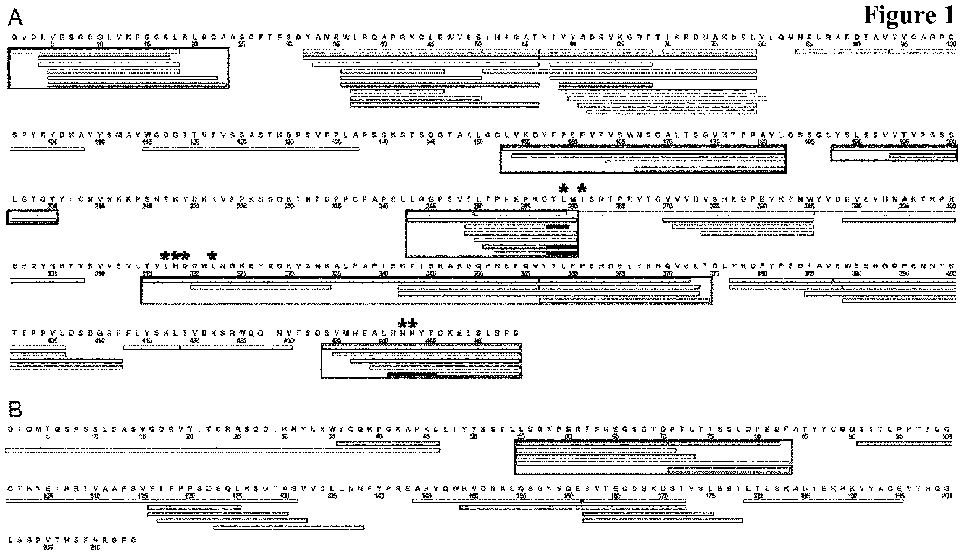

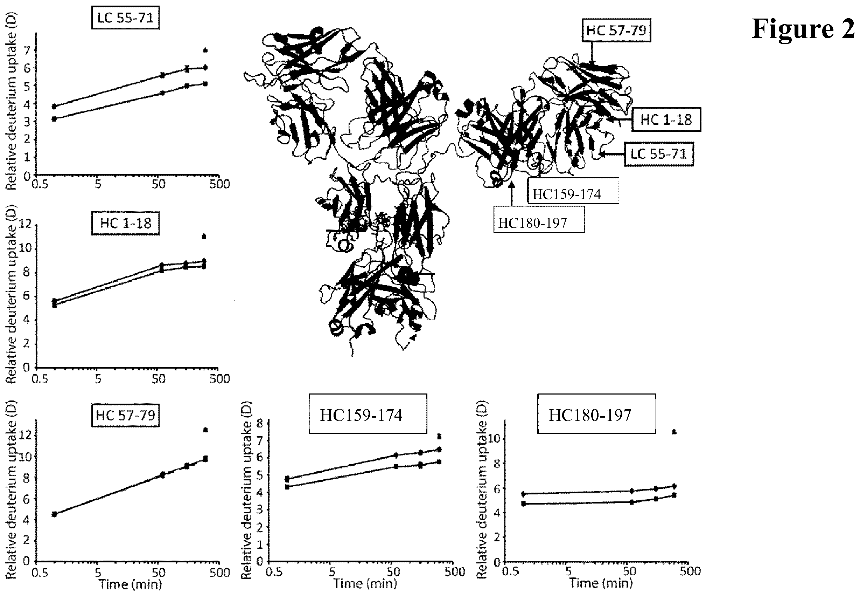

The invention is at least in part based on the finding that several regions of a monoclonal antibody in both the Fc-region and the Fab fragment show a reduction in deuterium uptake upon binding to FcRn (see Figure 1 and 2). These regions include residues 1-23, 145-174 (amino acid sequence GGTAALGCLVKDYFPEPVTVSWNSGALTSGVHTFPAVL, SEQ ID NO: 11), 180-197 (amino acid sequence YSLSSVVTVPSSSLGTQT, SEQ ID NO: 13) in the heavy chain as well as the residues 55-83 in the light chain (Kabat (variable domain) and EU index (constant region) numbering, respectively).

### I. DEFINITIONS

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype and the Kabat EU index numbering system (see pages 661-723) is used for the constant heavy chain domains (CHI, Hinge, CH2 and CH3).

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence alterations. In some embodiments, the number of amino acid alterations are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "alteration" denotes the mutation (substitution), insertion (addition), or deletion of one or more amino acid residues in a parent antibody or fusion polypeptide, e.g. a fusion polypeptide comprising at least an FcRn binding portion of an Fc-region, to obtain a modified antibody or fusion polypeptide. The term "mutation" denotes that the specified amino acid residue is substituted for a different amino acid residue. For example the mutation L234A denotes that the amino acid residue lysine at position 234 in an antibody Fc-region (polypeptide) is substituted by the amino acid residue alanine (substitution of lysine with alanine) (numbering according to the Kabat EU index numbering system).

A "naturally occurring amino acid residues" denotes an amino acid residue from the group consisting of alanine (three letter code: Ala, one letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophane (Trp, W), tyrosine (Tyr, Y), and valine (Val, V).

The term "amino acid mutation" denotes the substitution of at least one existing amino acid residue with another different amino acid residue (= replacing amino acid residue). The replacing amino acid residue may be a "naturally occurring amino acid residues" and selected from the group consisting of alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V). The replacing amino acid residue may be a "non-naturally occurring amino acid residue". See e.g. US 6,586,207, WO 98/48032, WO 03/073238, US 2004/0214988, WO 2005/35727, WO 2005/74524, Chin, J.W., et al., J. Am. Chem. Soc. 124 (2002) 9026-9027; Chin, J.W. and Schultz, P.G., ChemBioChem 11 (2002) 1135-1137; Chin, J.W., et al., PICAS United States of America 99 (2002) 11020-11024; and, Wang, L. and Schultz, P.G., Chem. (2002) 1-10 (all entirely incorporated by reference herein).

The term "amino acid insertion" denotes the (additional) incorporation of at least one amino acid residue at a predetermined position in an amino acid sequence. In one embodiment the insertion will be the insertion of one or two amino acid residues. The inserted amino acid residue(s) can be any naturally occurring or non-naturally occurring amino acid residue.

The term "amino acid deletion" denotes the removal of at least one amino acid residue at a predetermined position in an amino acid sequence.

The term "antibody" herein is used in the broadest sense and encompasses various antibody structures, including but not limited to monoclonal antibodies, multispecific antibodies (e.g. bispecific antibodies, trispecific antibodies), and antibody fragments so long as they exhibit the desired antigen-, and/or protein A and/or FcRn-binding activity.

The term "heterodimeric Fc-region" denotes an Fc-region that consists of two polypeptide chains that have different amino acid residues at corresponding positions, whereby the positions are determined according to the Kabat EU index numbering system, whereby the different positions affect the formation of heterodimers. Examples of such differences are the so-called "knobs into holes" substitutions (see, e.g., US 7,695,936 and US 2003/0078385). The following knobs and holes substitutions in the individual polypeptide chains of an Fc-region of an IgG antibody of subclass IgG1 have been found to increase heterodimer formation: 1) Y407T in one chain and T366Y in the other chain; 2) Y407A in one chain and T366W in the other chain; 3) F405A in one chain and T394W in the other chain; 4) F405W in one chain and T394S in the other chain; 5) Y407T in one chain and T366Y in the other chain; 6) T366Y and F405A in one chain and T394W and Y407T in the other chain; 7) T366W and F405W in one chain and T394S and Y407A in the other chain; 8) F405W and Y407A in one chain and T366W and T394S in the other chain; and 9) T366W in one chain and T366S, L368A, and Y407V in the other chain, whereby the last listed is especially suited. In addition, changes creating new disulfide bridges between the two Fc-region polypeptide chains facilitate heterodimer formation (see, e.g., US 2003/0078385). The following substitutions resulting in appropriately spaced apart cysteine residues for the formation of new intra-chain disulfide bonds in the individual polypeptide chains of an Fc-region of an IgG antibody of subclass IgG1 have been found to increase heterodimer formation: Y349C in one chain and S354C in the other; Y349C in one chain and E356C in the other; Y349C in one chain and E357C in the other; L351C in one chain and S354C in the other; T394C in one chain and E397C in the other; or D399C in one chain and K392C in the other. Further examples of heterodimerization facilitating amino acid changes are the so-called "charge pair substitutions" (see, e.g., WO 2009/089004). The following charge pair substitutions in the individual polypeptide chains of an Fc-region of an IgG antibody of subclass IgG1 have been found to increase heterodimer formation: 1) K409D or K409E in one chain and D399K or D399R in the other chain; 2) K392D or K392E in one chain and D399K or D399R in the other chain; 3) K439D or K439E in one chain and E356K or E356R in the other chain; 4) K370D or K370E in one chain and E357K or E357R in the other chain; 5) K409D and K360D in one chain plus D399K and E356K in the other chain; 6) K409D and K370D in one chain plus D399K and E357K in the other chain; 7) K409D and K392D in one chain plus D399K, E356K, and E357K in the other chain; 8) K409D and K392D in one chain and D399K in the other chain; 9) K409D and K392D in one chain and D399K and E356K in the other chain; 10) K409D and K392D in one chain and D399K and D357K in the other chain; 11) K409D and K370D in one chain and D399K and D357K in the other chain; 12) D399K in one chain and K409D and K360D in the other chain; and 13) K409D and K439D in one chain and D399K and E356K on the other.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "comparable length" denotes that two polypeptides comprise the identical number of amino acid residues or can be different in length by one or more and up to 10 amino acid residues at most. In one embodiment the (Fc-region) polypeptides comprise the identical number of amino acid residues or differ by a number of from 1 to 10 amino acid residues. In one embodiment the (Fc-region) polypeptides comprise the identical number of amino acid residues or differ by a number of from 1 to 5 amino acid residues. In one embodiment the (Fc-region) polypeptides comprise the identical number of amino acid residues or differ by a number of from 1 to 3 amino acid residues.

"Effector functions" refer to those biological activities attributable to the Fc-region of an antibody, which vary with the antibody class. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B-cell activation.

An "effective amount" of an agent, e.g., a pharmaceutical formulation, refers to an amount effective, at dosages and for periods of time necessary, to achieve the desired therapeutic or prophylactic result.

The term "Fc-region of human origin" denotes the C-terminal region of an immunoglobulin heavy chain of human origin that contains at least a part of the hinge region, the CH2 domain and the CH3 domain. In one embodiment, a human IgG heavy chain Fc-region extends from Cys226, or from Pro230, to the carboxyl-terminus of the heavy chain. In one embodiment the Fc-region has the amino acid sequence of SEQ ID NO: 02. However, the C-terminal lysine (Lys447) of the Fc-region may or may not be present.

The term "FcRn" denotes the human neonatal Fc-receptor. FcRn functions to salvage IgG from the lysosomal degradation pathway, resulting in reduced clearance and increased half-life. The FcRn is a heterodimeric protein consisting of two polypeptides: a 50 kDa class I major histocompatibility complex-like protein (a-FcRn) and a 15 kDa β2-microglobulin (β2m). FcRn binds with high affinity to the CH2-CH3 portion of the Fc-region of IgG. The interaction between IgG and FcRn is strictly pH dependent and occurs in a 1:2 stoichiometry, with one IgG binding to two FcRn molecules via its two heavy chains (Huber, A.H., et al., J. Mol. Biol. 230 (1993) 1077-1083). FcRn binding occurs in the endosome at acidic pH (pH < 6.5) and IgG is released at the neutral cell surface (pH of about 7.4). The pH-sensitive nature of the interaction facilitates the FcRn-mediated protection of IgGs pinocytosed into cells from intracellular degradation by binding to the receptor within the acidic environment of endosomes. FcRn then facilitates the recycling of IgG to the cell surface and subsequent release into the blood stream upon exposure of the FcRn-IgG complex to the neutral pH environment outside the cell.

The term "FcRn binding portion of an Fc-region" denotes the part of an antibody heavy chain polypeptide that extends approximately from EU position 243 to EU position 261 and approximately from EU position 275 to EU position 293 and approximately from EU position 302 to EU position 319 and approximately from EU position 336 to EU position 348 and approximately from EU position 367 to EU position 393 and EU position 408 and approximately from EU position 424 to EU position 440. In one embodiment one or more of the following amino acid residues according to the EU numbering of Kabat are altered F243, P244, P245 P, K246, P247, K248, D249, T250, L251, M252, 1253, S254, R255, T256, P257, E258, V259, T260, C261, F275, N276, W277, Y278, V279, D280, V282, E283, V284, H285, N286, A287, K288, T289, K290, P291, R292, E293, V302, V303, S304, V305, L306, T307, V308, L309, H310, Q311, D312, W313, L314, N315, G316, K317, E318, Y319, 1336, S337, K338, A339, K340, G341, Q342, P343, R344, E345, P346, Q347, V348, C367, V369, F372, Y373, P374, S375, D376, 1377, A378, V379, E380, W381, E382, S383, N384, G385, Q386, P387, E388, N389, Y391, T393, S408, S424, C425, S426, V427, M428, H429, E430, A431, L432, H433, N434, H435, Y436, T437, Q438, K439, and S440 (EU numbering).

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "full length antibody" denotes an antibody having a structure substantially similar to a native antibody structure comprising four polypeptides or having heavy chains that contain an Fc-region as defined herein. A full length antibody may comprise further domains, such as e.g. a scFv or a scFab conjugated to one or more of the chains of the full length antibody. These conjugates are also encompassed by the term full length antibody.

The term "dimeric polypeptide" denotes a complex comprising at least two polypeptides that are associated covalently. The complex may comprise further polypeptides that are also associated covalently or non-covalently with the other polypeptides. In one embodiment the dimeric polypeptide comprises two or four polypeptides.

The terms "heterodimer" or "heterodimeric" denote a molecule that comprises two polypeptides (e.g. of comparable length), wherein the two polypeptides have an amino acid sequence that have at least one different amino acid residue in a corresponding position, whereby corresponding position is determined according to the Kabat EU index numbering system.

The terms "homodimer" and "homodimeric" denote a molecule that comprises two polypeptides of comparable length, wherein the two polypeptides have an amino acid sequence that is identical in corresponding positions, whereby corresponding positions are determined according to the Kabat EU index numbering system.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A. et al., Sequences of Proteins of Immunological Interest, 5th ed., Bethesda MD (1991), NIH Publication 91-3242, Vols. 1-3. In one embodiment, for the VL, the subgroup is subgroup kappa I as in Kabat et al., supra. In one embodiment, for the VH, the subgroup is subgroup III as in Kabat et al., supra.

The term "derived from" denotes that an amino acid sequence is derived from a parent amino acid sequence by introducing alterations at at least one position. Thus a derived amino acid sequence differs from the corresponding parent amino acid sequence at at least one corresponding position (numbering according to Kabat EU index for antibody Fc-regions). In one embodiment an amino acid sequence derived from a parent amino acid sequence differs by one to fifteen amino acid residues at corresponding positions. In one embodiment an amino acid sequence derived from a parent amino acid sequence differs by one to ten amino acid residues at corresponding positions. In one embodiment an amino acid sequence derived from a parent amino acid sequence differs by one to six amino acid residues at corresponding positions. Likewise a derived amino acid sequence has a high amino acid sequence identity to its parent amino acid sequence. In one embodiment an amino acid sequence derived from a parent amino acid sequence has 80 % or more amino acid sequence identity. In one embodiment an amino acid sequence derived from a parent amino acid sequence has 90 % or more amino acid sequence identity. In one embodiment an amino acid sequence derived from a parent amino acid sequence has 95 % or more amino acid sequence identity.

The term "human Fc-region polypeptide" denotes an amino acid sequence which is identical to a "native" or "wild-type" human Fc-region polypeptide. The term "variant (human) Fc-region polypeptide" denotes an amino acid sequence which derived from a "native" or "wild-type" human Fc-region polypeptide by virtue of at least one "amino acid alteration". A "human Fc-region" is consisting of two human Fc-region polypeptides. A "variant (human) Fc-region" is consisting of two Fc-region polypeptides, whereby both can be variant (human) Fc-region polypeptides or one is a human Fc-region polypeptide and the other is a variant (human) Fc-region polypeptide.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., the CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence ("complementarity determining regions" or "CDRs") and form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3). HVRs as denoted herein include
(a) hypervariable loops occurring at amino acid residues 26-32 (L1), 50-52 (L2), 91-96 (L3), 26-32 (HI), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (HI), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to the Kabat EU index numbering system (Kabat et al., *supra*).

An "isolated" antibody is one which has been separated from a component of its natural environment. In some embodiments, an antibody is purified to greater than 95 % or 99 % purity as determined by, for example, electrophoretic (e.g., SDS-PAGE, isoelectric focusing (IEF), capillary electrophoresis) or chromatographic (e.g., size exclusion chromatography, ion exchange or reverse phase HPLC). For review of methods for assessment of antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "recombinant antibody" as used herein, denotes all antibodies (chimeric, humanized and human) that are prepared, expressed, created or isolated by recombinant means. This includes antibodies isolated from a host cell such as a NS0 or CHO cell, or from an animal (e.g. a mouse) that is transgenic for human immunoglobulin genes, or antibodies expressed using a recombinant expression vector transfected into a host cell. Such recombinant antibodies have variable and constant regions in a rearranged form. The recombinant antibodies can be subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The term "valent" as used within the current application denotes the presence of a specified number of binding sites in a (antibody) molecule. As such, the terms "bivalent", "tetravalent", and "hexavalent" denote the presence of two binding site, four binding sites, and six binding sites, respectively, in a (antibody) molecule. The bispecific antibodies as reported herein are in one preferred embodiment "bivalent".

The term "variable region" or "variable domain" refer to the domain of an antibody heavy or light chain that is involved in binding of the antibody to its antigen. The variable domains of the heavy chain and light chain (VH and VL, respectively) of an antibody generally have similar structures, with each domain comprising four framework regions (FRs) and three hypervariable regions (HVRs) (see, e.g., Kindt, T.J. et al. Kuby Immunology, 6th ed., W.H. Freeman and Co., N.Y. (2007), page 91). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S. et al., J. Immunol. 150 (1993) 880-887; Clackson, T. et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### II. THE CURRENT INVENTION

The neonatal Fc-receptor (FcRn) is important for the metabolic fate of antibodies of the IgG class in vivo. The FcRn functions to salvage wild-type IgG from the lysosomal degradation pathway, resulting in reduced clearance and increased half-life. It is a heterodimeric protein consisting of two polypeptides: a 50 kDa class I major histocompatibility complex-like protein (a-FcRn) and a 15 kDa β2-microglobulin (β2m). FcRn binds with high affinity to the CH2-CH3 portion of the Fc-region of an antibody of the class IgG. The interaction between an antibody of the class IgG and the FcRn is pH dependent and occurs in a 1:2 stoichiometry, i.e. one IgG antibody molecule can interact with two FcRn molecules via its two heavy chain Fc-region polypeptides (see e.g. Huber, A.H., et al., J. Mol. Biol. 230 (1993) 1077-1083).

Thus, an IgGs in vitro FcRn binding properties/characteristics are indicative of its in vivo pharmacokinetic properties in the blood circulation.

In the interaction between the FcRn and the Fc-region of an antibody of the IgG class different amino acid residues of the heavy chain CH2- and CH3-domain are participating. The amino acid residues interacting with the FcRn are located approximately between EU position 243 and EU position 261, approximately between EU position 275 and EU position 293, approximately between EU position 302 and EU position 319, approximately between EU position 336 and EU position 348, approximately between EU position 367 and EU position 393, at EU position 408, and approximately between EU position 424 and EU position 440. More specifically the following amino acid residues according to the EU numbering of Kabat are involved in the interaction between the Fc-region and the FcRn: F243, P244, P245 P, K246, P247, K248, D249, T250, L251, M252, 1253, S254, R255, T256, P257, E258, V259, T260, C261, F275, N276, W277, Y278, V279, D280, V282, E283, V284, H285, N286, A287, K288, T289, K290, P291, R292, E293, V302, V303, S304, V305, L306, T307, V308, L309, H310, Q311, D312, W313, L314, N315, G316, K317, E318, Y319, 1336, S337, K338, A339, K340, G341, Q342, P343, R344, E345, P346, Q347, V348, C367, V369, F372, Y373, P374, S375, D376, 1377, A378, V379, E380, W381, E382, S383, N384, G385, Q386, P387, E388, N389, Y391, T393, S408, S424, C425, S426, V427, M428, H429, E430, A431, L432, H433, N434, H435, Y436, T437, Q438, K439, and S440.

Site-directed mutagenesis studies have proven that the critical binding sites in the Fc-region of IgGs for FcRn are Histidine 310, Histidine 435, and Isoleucine 253 and to a lesser extent Histidine 433 and Tyrosine 436 (see e.g. Kim, J.K., et al., Eur. J. Immunol. 29 (1999) 2819-2825; Raghavan, M., et al., Biochem. 34 (1995) 14649-14657; Medesan, C., et al., J Immunol. 158 (1997) 2211-2217).

Methods to increase IgG binding to FcRn have been performed by mutating IgG at various amino acid residues: Threonine 250, Methionine 252, Serine 254, Threonine 256, Threonine 307, Glutamic acid 380, Methionine 428, Histidine 433, and Asparagine 434 (see Kuo, T.T., et al., J. Clin. Immunol. 30 (2010) 777-789).

In some cases antibodies with reduced half-life in the blood circulation are desired. For example, drugs for intravitreal application should have a long half-live in the eye and a short half-life in the blood circulation of the patient. Such antibodies also have the advantage of increased exposure to a disease site, e.g. in the eye.

Different mutations that influence the FcRn binding and therewith the half-live in the blood circulation are known. Fc-region residues critical to the mouse Fc-region-mouse FcRn interaction have been identified by site-directed mutagenesis (see e.g. Dall'Acqua, W.F., et al. J. Immunol 169 (2002) 5171-5180). Residues 1253, H310, H433, N434, and H435 (EU numbering according to Kabat) are involved in the interaction (Medesan, C., et al., Eur. J. Immunol. 26 (1996) 2533-2536; Firan, M., et al., Int. Immunol. 13 (2001) 993-1002; Kim, J.K., et al., Eur. J. Immunol. 24 (1994) 542-548). Residues 1253, H310, and H435 were found to be critical for the interaction of human Fc with murine FcRn (Kim, J.K., et al., Eur. J. Immunol. 29 (1999) 2819-2855). Residues M252Y, S254T, T256E have been described by Dall'Acqua et al. to improve FcRn binding by protein-protein interaction studies (Dall'Acqua, W.F., et al. J. Biol. Chem. 281 (2006) 23514-23524). Studies of the human Fc-human FcRn complex have shown that residues 1253, S254, H435, and Y436 are crucial for the interaction (Firan, M., et al., Int. Immunol. 13 (2001) 993-1002; Shields, R.L., et al., J. Biol. Chem. 276 (2001) 6591-6604). In Yeung, Y.A., et al. (J. Immunol. 182 (2009) 7667-7671) various mutants of residues 248 to 259 and 301 to 317 and 376 to 382 and 424 to 437 have been reported and examined.

It has now been found that several regions of a monoclonal antibody in the Fab fragment show a reduction in deuterium uptake upon binding to FcRn (see Figure 1 and 2). These regions include the residues 1-23, 145-174, 180-197 in the heavy chain as well as the residues 55-83 in the light chain (Kabat (variable domain and EU index (constant region) numbering, respectively).

Thus, not only Fc-region amino acid residues contribute to the strength and therewith the tightness of the antibody-FcRn interaction but also residues located in the CH1-domain and in the VH/VL domain.

Based on this finding it now possible to provide new amino acid mutations and combination of amino acid mutations to tailor make the in vivo half-life of antibodies.

HDX-MS was used to map the sites of a full length IgG1 antibody involved in binding to human FcRn. The effective sequence coverage of the HDX-MS of the anti-digoxygenin antibody with FcRn was 82 % for both HC and LC and reports on the deuterium uptake of 89 peptides (see Figure 1).

Several regions of the antibody in both the Fc- and Fab domains show a reduction in deuterium uptake upon binding to FcRn (see Figure 1 and 2). These regions include the residues 1-23, 145-174, 180-197 in the heavy chain as well as the residues 55-83 in the light chain (Kabat (variable domain) and EU index (constant region) numbering, respectively).

In region 1-23 analysis of HDX in overlapping peptides of 5-18, 5-22 and 5-23 show that no additional protection is observed for the C-terminal of the extended peptides (19-23). Comparison of peptide 1-18 with other peptides (4-18, 5-18) is not performed as the difference in back-exchange of the fully deuterated sample is more than 15%. Thus, considering that in HDX-MS experiments deuterium on the first two N-terminal residues in peptides is lost prior to MS detection, reduced deuterium uptake by FcRn binding can be localized to residues 3-18.

Comparison of HDX in overlapping peptides (145-174, 146-174, 156-174, 159-174) in region 145-174 in the antibody CH1 domain and considering that in HDX-MS experiments deuterium on the first two N-terminal residues in peptides is lost prior to MS detection shows that the protection of HDX by FcRn binding can be localized to residues 161-174.

For region 180-197 the overlapping peptide 186-197 shows only about half the reduction in deuterium uptake upon FcRn binding as peptide 180-197. Thus, considering that in HDX-MS experiments deuterium on the first two N-terminal residues in peptides is lost prior to MS detection, protection of HDX by FcRn can be localized to residues 182-197.

In the LC several peptides in region 55-83 in the framework region 3 show reduced deuterium uptake upon FcRn binding. By analysis of overlapping peptides (55-71, 55-73, 55-83, 71-82, 71-83) and considering that in HDX-MS experiments deuterium on the first two N-terminal residues in peptides is lost prior to MS detection the binding interaction is mainly located to residues 57-71.

The reduced HD-exchange of these regions remote from the Fc-region upon FcRn binding show that these regions comprise amino acid residues that participate in the antibody-FcRn interaction.

The invention is a method for selecting a full length antibody comprising the following steps:
a) generating from a parent full length antibody a plurality of full length antibodies by randomizing one or more amino acid residues selected from the amino acid residues at positions 1-23 in the heavy chain variable domain (numbering according to Kabat), at positions 55-83 in the light chain variable domain (numbering according to Kabat), at positions 145-174 in the first heavy chain constant domain (numbering according to EU index) and at positions 180-197 in the first heavy chain constant domain (numbering according to EU index),
b) determining the binding strength of each of the full length antibodies from the plurality of antibodies to the human neonatal Fc receptor (FcRn), and
c) selecting a full length antibody from the plurality of full length antibodies that has a different binding strength to the FcRn than the parent full length antibody.

Without being bound by this theory it is assumed that after a first interaction between the Fc-region of the antibody and the FcRn has been established a second interaction between the Fab-part of the antibody and the FcRn forms. This second interaction modifies the strength of the first interaction as shown by the data presented herein.

In the HD exchanges data presented herein it has been found that a direct interaction of the Fab with FcRn based on a relative low amount of positive charges in the variable domain exists.

Furthermore as reported herein additional positive charges have been applied to Ustekinumab in certain areas of the Fab region resulting in a significantly improved FcRn interaction strength. This interaction increase has been found to not follow the pH-dependent binding behavior, due to a later elution in the pH gradient of the FcRn affinity column.

In many FcRn column retention time analysis it has been found that all IgG display an individual retention time profile depending on their Fab sequence and thereby their individual Fab charge pattern. This observation can be modified by applying a high salt content to the running buffer resolving the additional charge dependent interaction.

It has been found that it is possible to shield the Fab influence by target preincubation to the respective antibody.

Thus, it has been found and is presented herein that each antibody has an individual charge pattern in the Fab region that can influence the binding of the antibody to the FcRn.

If the antibody has already a very strong or even too strong binding to FcRn (as exemplified e.g. by an elution pH value on an FcRn affinity column close to or even above pH 7.4 or by an unexpectedly shortened in vivo half-life indespite the strong FcRn binding) the antibody-FcRn interaction can be modified by reducing the number of charges in the Fab region of the antibody. For example in an FcRn affinity chromatography as reported in the examples of the current patent application an antibody having an elution pH of 7.4 or higher is an antibody with too strong FcRn interaction. By reducing the number of charges in the Fab region (eventually together with Fc-region engineering if the Fab region engineering is not sufficient) the retention time can also be reduced effecting an elution pH of less than pH 7.4. Such an antibody benefits from the FcRn interaction by in increased in vivo retention time.

If the antibody has a weak binding to FcRn (as exemplified e.g. by an elution pH value on an FcRn affinity column well below pH 7.4) the antibody-FcRn interaction can be modified by increasing the number of charges in the Fab region of the antibody. For example in an FcRn affinity chromatography as reported in the examples of the current patent application an antibody having an elution pH of 6.5 is an antibody with weak FcRn interaction. By increasing the number of charges in the Fab region (eventually together with Fc-region engineering if the Fab region engineering is not sufficient) the retention time can also be increased effecting an elution pH of close to pH 7.4. Such an antibody benefits from the FcRn interaction by in increased in vivo retention time.

Normally the changed charges in the Fab region effect the overall positive charge, i.e. the number of positive charges is reduced or increased or alternatively the number of negative charges is increased or reduced.

As not all regions of the Fab region can interact with the FcRn that is bound to the Fc-region of the respective antibody it has been found that by modifying residues in the regions as outlined herein, i.e. residues in the region spanning positions 1-23 in the heavy chain variable domain (numbering according to Kabat), at positions in the region spanning positions 55-83 in the light chain variable domain (numbering according to Kabat), at positions in the region spanning positions 145-174 in the first heavy chain constant domain (numbering according to EU index) and at positions in the region spanning positions 180-197 in the first heavy chain constant domain (numbering according to EU index), the antibody-FcRn interaction can be modified.

Furthermore in case of a bispecific antibody each Fab region can be engineered individually as one antibody Fc-region can interact with two FcRn molecules resulting in more potential interaction and modification sites.

Thus, in the method as reported herein the introduced mutations in the regions as defined herein introduce, remove or modify positive charge patches. Such mutations are known to a person skilled in the art, such as (in one letter amino acid code) e.g. E to Q (introduction of positive charge), T to E (reduction of positive charge), A to D (reduction of positive charge), S to D (reduction of positive charge), G to E (reduction of positive charge), G to R (introduction of positive charge), or S to K (introduction of positive charge). At physiological pH value the amino acid residues arginine (R), lysine (K) and histidine (H) are predominantly protonated and therefore positively charged, whereas the amino acid residues aspartate (D) and glutamate (E) are predominantly deprotonated (i.e. negatively charged).

The invention has been exemplified in the following with an anti-digoxygenin antibody.

The following mutations have been introduced into an anti-digoxygenin antibody:
- HC-E6Q (variant 1),
- HC-T164E (variant 2),
- HC-A162D and HC-S165D (variant 3),
- HC-G194E (variant 4),
- HC-S191D and HC-Q196D (variant 5),
- LC-G57R (variant 6),
- LC-G57R and LC-S60K (variant 7),
- HC164E, HC-A162D, HC-S165D, HC-G194E, HC-S191D and HC-Q196D (variant 8),
- HC-E6Q, HC-A162D, HC-T164E, HC-S165D, HC-S191D, HC-G194E, HC-Q196D, LC-G57K and LC-S60K (variant 9).

The properties of these mutants on an FcRn affinity column and in an SPR-based binding assay are shown in the following Table (effect on antibody-FcRn interaction):

| **sample** | **relative FcRn retention time** | **relative SPR binding** |
|---|---|---|
| reference anti-digoxygenin antibody | 100 % (40 min.) | 100 % |
| variant 1 | increased | 107 % |
| variant 2 | reduced | 94 % |
| variant 3 | increased | 178 % |
| variant 4 | reduced | 96 % |
| variant 5 | increased | 167 % |
| variant 6 | increased | 126 % |
| variant 7 | increased | 161 % |
| variant 8 | reduced | 69 % |
| variant 9 | increased | 120 % |

Thus, it can be seen from the data presented above that by changing the charge pattern in the variable domain and/or the first constant domain (VH1 and CL) a change in the FcRn binding can be effected.

One aspect as reported herein is a plurality of variant full length antibodies generated from a single parent full length antibody by randomizing one or more amino acid residues selected from the amino acid residues at positions 1-23 in the heavy chain variable domain (numbering according to Kabat), at positions 55-83 in the light chain variable domain (numbering according to Kabat), at positions 145-174 in the first heavy chain constant domain (numbering according to EU index) and at positions 180-197 in the first heavy chain constant domain (numbering according to EU index).

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat) and at positions 181-196 in the first heavy chain constant domain (numbering according to EU index).

Another aspect as reported herein is the use of one or more amino acid mutations at positions selected from the group of positions comprising positions 1-23 in the heavy chain variable domain (numbering according to Kabat), positions 55-83 in the light chain variable domain (numbering according to Kabat), positions 145-174 in the first heavy chain constant domain (numbering according to EU index) and positions 180-197 in the first heavy chain constant domain (numbering according to EU index) for changing the in vivo half-life of a full length antibody.

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat) and at positions 181-196 in the first heavy chain constant domain (numbering according to EU index).

A further aspect as reported herein is a variant full length antibody comprising two light chain polypeptides and two heavy chain polypeptides, wherein the variant antibody is derived from a parent full length antibody by introducing amino acid mutations at one or more positions selected from the group of positions comprising positions 1-23 in the heavy chain variable domain (numbering according to Kabat), positions 55-83 in the light chain variable domain (numbering according to Kabat), positions 145-174 in the first heavy chain constant domain (numbering according to EU index) and positions 180-197 in the first heavy chain constant domain (numbering according to EU index), and wherein the variant antibody has a different affinity for the human neonatal Fc receptor than the parent full length antibody.

In one embodiment the one or more amino acid residues are selected from the amino acid residues at positions 3-18 in the heavy chain variable domain (numbering according to Kabat), at positions 57-71 in the light chain variable domain (numbering according to Kabat), at positions 161-174 in the first heavy chain constant domain (numbering according to EU index) and at positions 182-197 in the first heavy chain constant domain (numbering according to EU index).

In one embodiment the one or more amino acid residues are selected from the amino acid residues at positions 5-18 in the heavy chain variable domain (numbering according to Kabat), at positions 57-70 in the light chain variable domain (numbering according to Kabat) and at positions 181-196 in the first heavy chain constant domain (numbering according to EU index).

One aspect as reported herein is an antibody that has a mutation at one or more of the following amino acid residues
- residues 6, 162, 164, 165, 191, 194, 195 and 196 in the heavy chain
- residues 57 and 60 in the light chain
(numbering according to Kabat (variable domain) and EU index (constant region), respectively).

In one embodiment the antibody is a full length IgG antibody. In one embodiment the antibody is a full length IgG1 antibody.

In one embodiment the antibody is a bispecific antibody.

It has been found that the heavy chain peptide stretch comprising amino acid residues 3 to 18 can be modified to enlarge an existing positive surface charge patch which can interact with its negative counterpart on the FcRn. Only the E6Q mutation should be energetically tolerated.

In one embodiment the antibody has the amino acid mutation E6Q in the heavy chain variable domain.

It has been found that the heavy chain peptide stretch comprising amino acid residues 159 to 174 breaks two adjacent negative charge patches which are opposite a positive patch on the CH2 domain. Thus, an increased CH1-CH2 interaction can bring the Fab closer to FcRn which in turn might favor FcRn binding. This patch is best modified together with the heavy chain peptide stretch comprising amino acid residues 182-197 because both are in proximity of the same CH2 domain patch. Thus, one or more of the amino acid residues at positions 162 and/or 164 and/or 165 and/or 191 and/or 194 and/or 195 and/or 196 should be modified to an acidic residue, in one preferred embodiment to D or E, in order to strengthen antibody-FcRn binding/interaction.

In one embodiment the antibody has an acidic amino acid at one or more of the amino acid positions 162, 164, 165, 191, 194, 195 and 196 of the heavy chain first constant domain (CHI). In one embodiment the acidic amino acid is D or E. In on embodiment the antibody has an acidic amino acid residue at two or more of the amino acid positions 162, 164, 165, 191, 194, 195 and 196 of the heavy chain first constant domain (CHI) whereby the acidic amino acid residues are selected independently of each other.

It has been found that the peptide stretch comprising amino acid residues 57 to 71 of the light chain variable domain forms a weak positive patch opposite a negative patch on FcRn. Mutating positive residues in this stretch to negative residues, i.e. basic residues, can strengthen the antibody-FcRn interaction. In one preferred embodiment the amino acid residues at position 57 and/or 60 of the light chain variable domain are independently of each other a basic amino acid residue. In one embodiment the basic amino acid residue is selected from K and R.

In one embodiment the antibody has a basic amino acid residue at one or both of the amino acid positions 57 and 60 of the light chain variable domain. In one embodiment the basic amino acid residue is K or R. In one embodiment the antibody has a basic amino acid residue at both of the positions 57 and 60 of the light chain variable domain whereby the basic amino acid residues are selected independently of each other.

In one embodiment the antibody has one or more of the following amino acid mutations
- heavy chain E6Q, and/or
- heavy chain A162D, and/or
- heavy chain A162E, and/or
- heavy chain T164D, and/or
- heavy chain T164E, and/or
- heavy chain S165D, and/or
- heavy chain S165E, and/or
- heavy chain S191D, and/or
- heavy chain S191E, and/or
- heavy chain G194D, and/or
- heavy chain G194E, and/or
- heavy chain T195D, and/or
- heavy chain T195E, and/or
- heavy chain Q196D, and/or
- heavy chain Q196E, and/or
- light chain G57K, and/or
- light chain G57R, and/or
- light chain S60K, and/or
- light chain S60R.

In one aspect as reported herein an antibody may be selected by screening random libraries for antibodies with the desired antibody-FcRn interactions.

For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Patent publications describing human antibody phage libraries include, for example: US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

A screening system based on Vaccinia virus-mediated expression of whole antibodies in mammalian cells is reported in US 2002/0123057. Another screening system is based on cell surface expression of antibodies in mammalian cells (Ho, et al., Proc. Natl. Acad. Sci. USA 103 (2006) 9637-9642).

Cellular display is described by Higuchi et al. in COS cells (J. Immunol. Meth. 202 (1997) 193-204). Beerli et al. report a Sindbis virus-based scFv cell surface display library produced from antigen-specific B-cells in BHK cells (Proc. Natl. Acad. Sci. USA 105 (2008) 14336-14341). Ho and Pastan report methods with HEK293 cells (scFv) (Methods Mol. Biol. 562 (2009) 99-113). Lymphocyte display is reported by Alonso-Camino et al. (PLoS One. 4 (2009) e7174). Zhou et al. report methods using HEK293 cells (Acta Biochim. Biophys. Sin. 42 (2010) 575-584). Zhou et al. report the Flp-In system (MAbs. 2 (2010) 508-518).

Taube, R., et al report (PLOS One 3 (2008) e3181) stable expression of human antibodies on the surface of human cells and lentiviral virus particles.

In WO 2007/047578 the cell display of antibody libraries is reported.

The display and/or secretion of antibodies on eukaryotic cells is/are a further method(s) to isolate antibodies. These are generated by cloning antibody encoding nucleic acids into lentiviral vectors which are used to transduce cells to enable expression of the antibodies on the surface of these cells. The used vectors carry an antibody light chain, an antibody heavy chain, an alternatively spliced membrane anchor, or tag, or fluorescent marker protein.

Sasaki-Haraguchi, N., et al. (Biochem. Biophys. Res. Commun. 423 (2012) 289-294) report about mechanistic insights into human pre-mRNA splicing of human ultra-short introns: potential unusual mechanism identifies G-rich introns.

In one embodiment the method/display system as reported in WO 2013/092720 using a lentiviral expression library in combination with a lentiviral expression vector comprising an EV71-IRES linked bicistronic expression cassette for the expression of a full length antibody light chain and a full length antibody heavy chain in a soluble as well as membrane bound form is employed in the method as reported herein. The used a lentiviral vector includes the viral long terminal repeats 3'LTR and 5'LTR, an antibody light chain, an EV71 IRES, an antibody heavy chain.

In one embodiment the method/display system as reported in EP 13178581.8 is used.

In certain embodiments, an antibody provided herein is a multispecific antibody, e.g. a bispecific antibody. Multispecific antibodies are monoclonal antibodies that have binding specificities for at least two different sites/antigens.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

Engineered antibodies with three or more functional antigen binding sites, including "Octopus antibodies", are also included herein (see, e.g. US 2006/0025576).

The antibody herein also includes a "Dual Acting Fab" or "DAF" (see, US 2008/0069820, for example).

The antibody herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793.

In certain embodiments, antibody variants/mutants having one or more amino acid mutations are provided. Conservative mutations are shown in the following Table under the heading of "preferred mutations". More substantial changes are provided in the following Table under the heading of "exemplary mutations", and as further described below in reference to amino acid side chain classes. Amino acid mutations may be introduced into an antibody of interest and the products screened for a desired FcRn binding.

**TABLE**

| **Original Residue** | **Exemplary Mutations** | **Preferred Mutations** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative mutations will entail exchanging a member of one of these classes for another class.

One type of mutational variant involves mutating one or more residues of a parent antibody (e.g. a humanized or human antibody). Generally, the resulting variant(s) selected for further study will have modifications (e.g., improvements) in FcRn binding properties relative to the parent antibody and will have substantially retained the other biological properties of the parent antibody.

In some embodiments the mutations are introduced into the encoding nucleic acid by any of a variety of methods (e.g., error-prone PCR, chain shuffling, or oligonucleotide-directed mutagenesis). A library is then created. The library is then screened to identify any antibody variants with the desired FcRn affinity. Another method to introduce diversity involves position-directed approaches, in which several amino acid residues (e.g., 4-6 residues at a time) are randomized.

In one embodiment the mutation results in the change of the mutated amino acid residue to a different residue of the same group.

In one embodiment the mutation results in the change of the mutated amino acid residue to a different residue from a different group.

In certain embodiments, one or more amino acid modifications may be introduced into the Fc-region of an antibody provided herein, thereby generating an Fc-region variant. The Fc-region variant may comprise a human Fc-region sequence (e.g., a human IgG1, IgG2, IgG3 or IgG4 Fc-region) comprising an amino acid modification (e.g. a substitution) at one or more amino acid positions.

In certain embodiments, the disclosure contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express FcγRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of *in vitro* assays to assess ADCC activity of a molecule of interest is described in US 5,500,362 (see, e.g. Hellstrom, I. et al., Proc. Natl. Acad. Sci. USA 83 (1986) 7059-7063; and Hellstrom, I. et al., Proc. Natl. Acad. Sci. USA 82 (1985) 1499-1502); US 5,821,337 (see Bruggemann, M. et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes, R. et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H. et al., J. Immunol. Methods 202 (1996) 163-171; Cragg, M.S. et al., Blood 101 (2003) 1045-1052; and Cragg, M.S. and M.J. Glennie, Blood 103 (2004) 2738-2743). FcRn binding and *in vivo* clearance/half-life determinations can also be performed using methods known in the art (see, e.g., Petkova, S.B. et al., Int. Immunol. 18 (2006: 1759-1769).

Antibodies with reduced effector function include those with substitution of one or more of Fc-region residues 238, 265, 269, 270, 297, 327 and 329 (US 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US 7,332,581).

Certain antibody variants with improved or diminished binding to FcRs are described. (See, e.g., US 6,737,056; WO 2004/056312, and Shields, R.L. et al., J. Biol. Chem. 276 (2001) 6591-6604)

In certain embodiments, an antibody variant comprises an Fc-region with one or more amino acid substitutions which improve ADCC, e.g., substitutions at positions 298, 333, and/or 334 of the Fc-region (EU numbering of residues).

In some embodiments, alterations are made in the Fc-region that result in altered (*i.e*., either improved or diminished) C1q binding and/or Complement Dependent Cytotoxicity (CDC), e.g., as described in US 6,194,551, WO 99/51642, and Idusogie, E.E. et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc-region with one or more substitutions therein which improve binding of the Fc-region to FcRn. Such Fc variants include those with substitutions at one or more of Fc-region residues: 238, 256, 265, 272, 286, 303, 305, 307, 311, 312, 317, 340, 356, 360, 362, 376, 378, 380, 382, 413, 424 or 434, e.g., substitution of Fc-region residue 434 (US 7,371,826).

See also Duncan, A.R. and Winter, G., Nature 322 (1988) 738-740; US 5,648,260; US 5,624,821; and WO 94/29351 concerning other examples of Fc-region variants.

### The teaching of the current invention

Herein it has been found that the modification of certain residues within the VL/CH1 domain of an antibody can be used to influence the antibody-FcRn interaction.

### Recombinant Methods

Antibodies may be produced using recombinant methods and compositions, e.g., as described in US 4,816,567. In one embodiment, isolated nucleic acid encoding an antibody as described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of producing an antibody as reported herein is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US 5,959,177, US 6,040,498, US 6,420,548, US 7,125,978, and US 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### Description of the Figures

- **Figure 1**: HDX-MS peptide map of the anti-digoxygenin antibody. Peptic peptides from which HDX data could be obtained are shown as white bars for the HC (A) and LC (B). Open big boxes denote peptides with reduced deuterium content upon FcRn binding. Solid boxes indicate targeted regions where ETD of deuterated peptic peptides was used to resolve differential deuterium labeling upon FcRn binding to individual sites. Antibody residues implicated in FcRn binding in crystal structures of rat Fc-FcRn and human FcRn-Fc-YTE are indicated with asterisks (see Martin, W.L., et al., Mol. Cell. 7 (2001) 867-877, Oganesyan, V., et al., J. Biol. Chem. 289 (2014) 7812-7824).
- **Figure 2**: HDX profiles of anti-digoxygenin antibody regions in the presence and absence of human FcRn. HDX plots are shown for light chain peptide 55-71 and heavy chain peptides 1-18, 57-79, 159-174 and 180-197. Upper curves display the HDX of the antibody alone and lower curves display HDX of HC and LC peptides, respectively, in the presence of FcRn. For HC 57-79 with FcRn is shown as a dashed black curve. The deuterium incorporation was monitored in triplicates at 1 min, 1 h, 2.5 h and 5 h. The full deuterium level measured in control experiments (at 90% D2O) is shown in black at the 5 h time point.

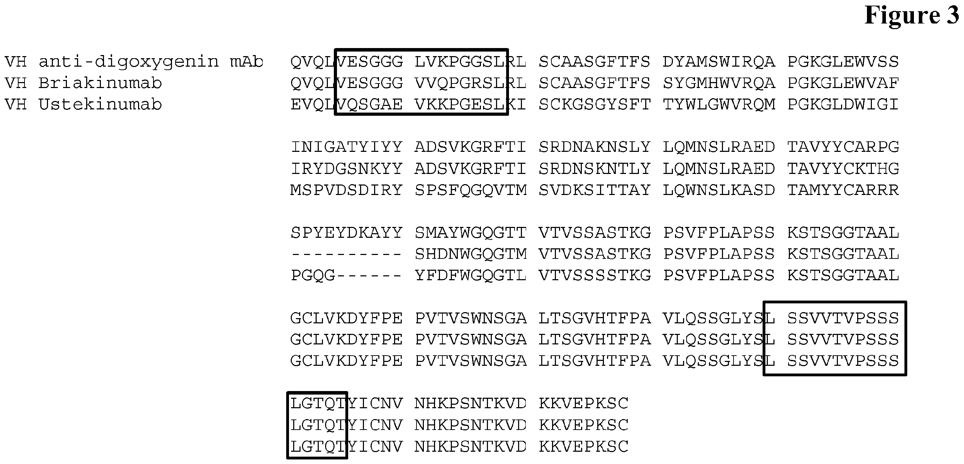

- **Figure 3**: Alignment of the amino acid sequence of the heavy chain variable domains of the anti-digoxigenin antibody, Briakinumab and Ustekinumab. Amino acid residue stretches in which for all three antibodies protection from HDX has been found are boxed.

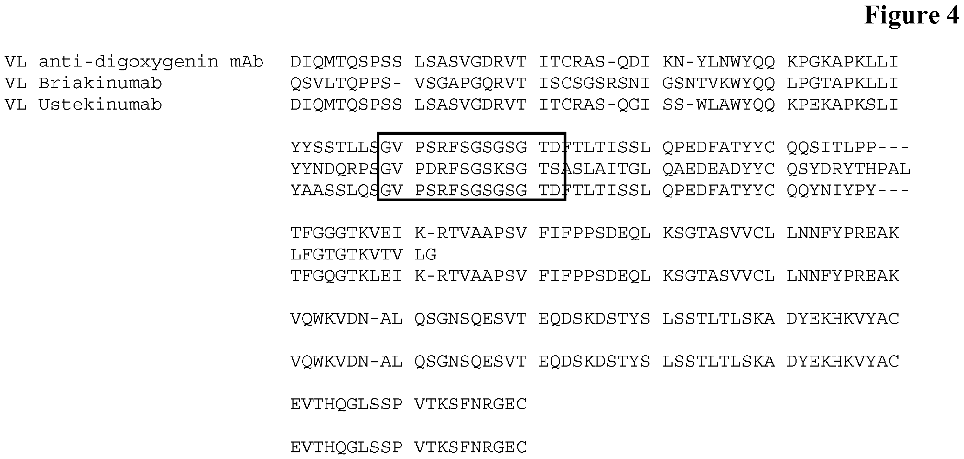

- **Figure 4**: Alignment of the amino acid sequence of the light chain variable domains of the anti-digoxigenin antibody, Briakinumab and Ustekinumab. Amino acid residue stretches in which for all three antibodies protection from HDX has been found are boxed.

The following Examples, Sequences and Figures are provided to aid the understanding of the present invention, the true scope of which is set forth in the appended claims. It is understood that modifications can be made in the procedures set forth without departing from the spirit of the invention.

### Examples

### Materials and Methods

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

### Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany)

### Reagents

All commercial chemicals, antibodies and kits were used as provided according to the manufacturer's protocol if not stated otherwise.

### Example 1

### Generation of recombinant expression vectors for the anti-digoxygenin antibody

### Generation of vector for the expression of the anti-digoxygenin antibody heavy chain

The heavy chain encoding fusion gene comprising the human IgG1 constant region (CH1, hinge, CH2, CH3) and the anti-digoxygenin antibody heavy chain variable domain was assembled by fusing a DNA fragment coding for the respective anti-digoxygenin antibody heavy chain variable domain to a sequence element coding the human IgG1 constant region.

The anti-digoxygenin antibody heavy chain variable domain has the following amino acid sequence:

The human IgG1 constant region has the following amino acid sequence:

The expression vector also comprised an origin of replication from the vector pUC18, which allows replication of this plasmid in E. coli, and a beta-lactamase gene, which confers ampicillin resistance in E. coli.

The transcription unit of the antibody heavy chain comprises the following functional elements in 5' to 3' direction:
- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- a heavy chain variable (VH) domain encoding nucleic acid,
- a human IgG1 constant region encoding nucleic acid, and
- the bovine growth hormone polyadenylation sequence (BGH pA).

### Generation of vector for the expression of the anti-digoxygenin antibody light chain

The kappa light chain encoding fusion gene comprising the human Ig-kappa constant region (CL-kappa) and the anti-digoxygenin antibody light chain variable domain of the kappa isotype was assembled by fusing a DNA fragment coding for the respective anti-digoxygenin antibody light chain variable domain to a sequence element coding for the human Ig-kappa constant region.

The anti-digoxygenin antibody light chain variable domain has the following amino acid sequence:

The human Ig-kappa constant region has the following amino acid sequence:

The expression vector also comprised an origin of replication from the vector pUC18, which allows replication of this plasmid in E. coli, and a beta-lactamase gene which confers ampicillin resistance in E. coli.

The transcription unit of the antibody kappa light chain comprises the following functional elements in 5' to 3' direction:
- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- a light chain variable (VL) domain encoding nucleic acid,
- a human Ig-kappa constant region encoding nucleic acid, and
- the bovine growth hormone polyadenylation sequence (BGH pA).

### Example 2

### Recombinant production of the anti-digoxygenin antibody

The antibody was produced in transiently transfected HEK293 cells (human embryonic kidney cell line 293-derived) cultivated in F17 Medium (Invitrogen Corp.). For transfection of the respective vectors as described in Example 1 the "293-Free" Transfection Reagent (Novagen) was used. The antibody was expressed from individual expression plasmids. Transfections were performed as specified in the manufacturer's instructions. Recombinant antibody-containing cell culture supernatants were harvested three to seven days after transfection. Supernatants were stored at reduced temperature (e.g. -80 °C) until purification.

General information regarding the recombinant expression of human immunoglobulins in e.g. HEK293 cells is given in: Meissner, P. et al., Biotechnol. Bioeng. 75 (2001) 197-203.

### Example 3

### Purification of recombinant anti-digoxygenin antibody

The antibody-containing culture supernatants were filtered and purified by two chromatographic steps.

The antibody was captured by affinity chromatography using HiTrap MabSelectSuRe (GE Healthcare) equilibrated with PBS (1 mM KH₂PO₄, 10 mM Na₂HPO₄, 137 mM NaCl, 2.7 mM KCl), pH 7.4. Unbound proteins were removed by washing with equilibration buffer, and the antibody was recovered with 25 mM citrate buffer, pH 3.1, which was immediately after elution adjusted to pH 6.0 with 1 M Tris-base, pH 9.0.

Size exclusion chromatography on Superdex 200™ (GE Healthcare) was used as second purification step. The size exclusion chromatography was performed in 20 mM histidine buffer, 0.14 M NaCl, pH 6.0. The antibody containing solutions were concentrated with an Ultrafree-CL centrifugal filter unit equipped with a Biomax-SK membrane (Millipore, Billerica, MA, USA) and stored at -80 °C.

### Example 4

### Generation of recombinant expression vectors for Ustekinumab and Briakinumab

Ustekinumab: CNTO 1275, Stelara™, CAS Registry Number 815610-63-0
Briakinumab: ABT 874, J 695, Ozespa™, SEQ ID NO: 36, WO2001/014162

For the expression of the above antibodies expression plasmids for transient expression (e.g. in HEK293-F) based either on a cDNA organization with or without a CMV-Intron A promoter or on a genomic organization with a CMV promoter were used.

Beside the antibody expression cassette the plasmids contained:
- an origin of replication which allows replication of this plasmid in *E. coli,*
- a β-lactamase gene which confers ampicillin resistance in *E. coli.,* and
- the dihydrofolate reductase gene from *Mus musculus* as a selectable marker in eukaryotic cells.

The transcription unit of the antibody gene was composed of the following elements:
- unique restriction site(s) at the 5' end
- the immediate early enhancer and promoter from the human cytomegalovirus,
- followed by the Intron A sequence in the case of the cDNA organization,
- a 5 '-untranslated region of a human antibody gene,
- an immunoglobulin heavy chain signal sequence,
- the human antibody chain either as cDNA or as genomic organization with the immunoglobulin exon-intron organization
- a 3' non-translated region with a polyadenylation signal sequence, and
- unique restriction site(s) at the 3' end.

The fusion genes comprising the antibody chains were generated by PCR and/or gene synthesis and assembled by known recombinant methods and techniques by connection of the according nucleic acid segments *e.g.* using unique restriction sites in the respective plasmids. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Example 5

### Recombinant production of Ustekinumab and Briakinumab

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

The antibodies were generated by transient transfection with the respective plasmids (e.g. encoding the heavy chain, as well as the corresponding light chain) using the HEK293-F system (Invitrogen) according to the manufacturer's instruction. Briefly, HEK293-F cells (Invitrogen) growing in suspension either in a shake flask or in a stirred fermenter in serum-free FreeStyle™ 293 expression medium (Invitrogen) were transfected with a mix of the respective expression plasmids and 293fectin™ or fectin (Invitrogen). For 2 L shake flask (Corning) HEK293-F cells were seeded at a density of 1*10⁶ cells/mL in 600 mL and incubated at 120 rpm, 8 % CO₂. The day after the cells were transfected at a cell density of ca. 1.5*10⁶ cells/mL with ca. 42 mL mix of A) 20 mL Opti-MEM (Invitrogen) with 600 µg total plasmid DNA (1 µg/mL) encoding the heavy chain, respectively and the corresponding light chain in an equimolar ratio and B) 20 ml Opti-MEM + 1.2 mL 293 fectin or fectin (2 µL/mL). According to the glucose consumption glucose solution was added during the course of the fermentation. The supernatant containing the secreted antibody was harvested after 5-10 days and antibodies were either directly purified from the supernatant or the supernatant was frozen and stored.

### Example 6

### Purification of recombinant Ustekinumab and Briakinumab

The antibodies were purified from cell culture supernatants by affinity chromatography using MabSelectSure-Sepharose™ (GE Healthcare, Sweden), hydrophobic interaction chromatography using butyl-Sepharose (GE Healthcare, Sweden) and Superdex 200 size exclusion (GE Healthcare, Sweden) chromatography.

Briefly, sterile filtered cell culture supernatants were captured on a MabSelectSuRe resin equilibrated with PBS buffer (10 mM Na₂HPO₄, 1 mM KH₂PO₄, 137 mM NaCl and 2.7 mM KCl, pH 7.4), washed with equilibration buffer and eluted with 25 mM sodium citrate at pH 3.0. The eluted antibody fractions were pooled and neutralized with 2 M Tris, pH 9.0. The antibody pools were prepared for hydrophobic interaction chromatography by adding 1.6 M ammonium sulfate solution to a final concentration of 0.8 M ammonium sulfate and the pH adjusted to pH 5.0 using acetic acid. After equilibration of the butyl-Sepharose resin with 35 mM sodium acetate, 0.8 M ammonium sulfate, pH 5.0, the antibodies were applied to the resin, washed with equilibration buffer and eluted with a linear gradient to 35 mM sodium acetate pH 5.0. The antibody containing fractions were pooled and further purified by size exclusion chromatography using a Superdex 200 26/60 GL (GE Healthcare, Sweden) column equilibrated with 20 mM histidine, 140 mM NaCl, pH 6.0. The antibody containing fractions were pooled, concentrated to the required concentration using Vivaspin ultrafiltration devices (Sartorius Stedim Biotech S.A., France) and stored at -80 °C.

**Table: Yields of the antibodies.**

| **Sample** | **Final purified product [mg]** | **Final purified product concentration [mg/mL]** |
|---|---|---|
| Briakinumab | 23.50 | 2.36 |
| Ustekinumab | 12.55 | 2.67 |

Purity and antibody integrity were analyzed after each purification step by CE-SDS using microfluidic Labchip technology (Caliper Life Science, USA). Five µl of protein solution was prepared for CE-SDS analysis using the HT Protein Express Reagent Kit according manufacturer's instructions and analyzed on LabChip GXII system using a HT Protein Express Chip. Data were analyzed using LabChip GX Software.

### Example 7

### Expression of FcRn in HEK293 cells

FcRn was transiently expressed by transfection of HEK293 cells with two plasmids containing the coding sequence of FcRn (SEQ ID NO: 09) and of beta-2-microglobulin (SEQ ID NO: 10). The transfected cells were cultured in shaker flasks at 36.5 °C, 120 rpm (shaker amplitude 5 cm), 80 % humidity and 7 % CO₂. The cells were diluted every 2 - 3 days to a density of 3 to 4*10⁵ cells/ml.

For transient expression, a 14 1 stainless steel bioreactor was started with a culture volume of 8 1 at 36.5°C, pH 7.0 ± 0.2, pO₂ 35 % (gassing with N₂ and air, total gas flow 200 ml min⁻¹) and a stirrer speed of 100 - 400 rpm. When the cell density reached 20*10⁵ cells/ml, 10 mg plasmid DNA (equimolar amounts of both plasmids) was diluted in 400 ml Opti-MEM (Invitrogen). 20 ml of 293fectin (Invitrogen) was added to this mixture, which was then incubated for 15 minutes at room temperature and subsequently transferred into the fermenter. From the next day on, the cells were supplied with nutrients in continuous mode: a feed solution was added at a rate of 500 ml per day and glucose as needed to keep the level above 2 g/l. The supernatant was harvested 7 days after transfection using a swing head centrifuge with 1 l buckets: 4000 rpm for 90 minutes. The supernatant (13 L) was cleared by a Sartobran P filter (0.45 µm + 0.2 µm, Sartorius) and the FcRn beta-2-microglobulin complex was purified therefrom.

### Example 8

### Characterization of monoclonal antibodies and FcRn by MS

Reduced intact mass spectrometry was performed on 50 µg monoclonal antibody or FcRn which was reduced and denatured using 0.5 M TCEP (Perbio, Bonn, Germany) in 4 M guanidinium hydrochloride solution at 37 °C for 30 minutes. Samples were desalted by size exclusion chromatography (Sephadex G-25, isocratic elution with 40 % acetonitrile with 2 % formic acid (v/v)). ESI Mass spectra were recorded on a Q-TOF instrument (MaXis, Bruker, Germany) equipped with a Triversa NanoMate (Advion, Ithaca, USA). For data evaluation, in house developed software was used.

Peptide mapping of monoclonal antibody and FcRn (250 µg) was done by denaturing the compounds by addition of 0.4 M Tris, 8 M guanidinium hydrochloride, pH 8 and 0.24 M DTT for one hour at 37 °C and alkylated by addition of 0.6 M iodoacetic acid in water for 15 minutes at room temperature in the dark. The samples were buffer exchanged to 50 mM Tris/HCl, pH 7.5 using NAP 5 Sephadex G-25 DNA grade columns (GE Healthcare, Munich, Germany). Digestion was performed with trypsin (Promega, Mannheim, Germany) for 5 hours at 37 °C (enzyme to substrate ratio of 1:37). The peptide mixture obtained was injected and separated without pretreatment using reversed phase U-HPLC (NanoAcquity, Waters GmbH, Eschborn, Germany). An Acquity UPLC BEH C18 column (1 x 150 mm, 1.7 µm particle diameter, 300 Å pore size) from Waters was used for separation. The solvents were 0.1 % (v/v) formic acid in water (A) and in acetonitrile (B) (Sigma Aldrich, Munich, Germany). A linear gradient of 60 µl/min. from 1 to 40 % B was run over 120 min. at 50 °C. Mass analysis was performed by coupling the UPLC system to a LTQ Orbitrap XL tandem mass spectrometer (Thermo Fisher Scientific, Dreieich, Germany) operating in positive ion mode through a Triversa NanoMate interface (Advion, Ithaca, USA).

### Example 9

### Hydrogen/deuterium exchange mass spectrometry (HDX-MS)

HDX-MS experiments were performed using the following sample setup:
The monoclonal antibody (73 pmol/µl) with and without FcRn (112 pmol/µl) were mixed and diluted in with D₂O (99.9 atom% deuterium) containing 50 mM sodium phosphate, 50 mM NaCl, pH 6.5 to a final deuterium content of 90 % and concentrations of antibody of 1.2 pmol/µl and FcRn of 8.96 pmol/µl (84 % antibody bound, 1:2 IgG1:FcRn binding ratio).

Theoretical calculations were based on a K_{D} of 0.6 µM for the IgG1-FcRn interaction and a final volume of 25 µl following dilution with D₂O. Following 15 min. pre-incubation of samples, deuterium labeling was initiated at room temperature for different time intervals: 0 min., 1 min., 1 hour, 2.5 hours and 5 hours. At each time interval aliquots (25 µl) of 30 pmol target protein was removed from the labeling mixture and quenched to a final pH of 2.5 in an ice cold mixture of 25 µl 50 mM Na phosphate, 50 mM NaCl, pH 6.5 and 50 µl 0.5 M TCEP, 6 M Guanidinium-hydrochloride in phosphoric acid, pH 2.3 and frozen to -80 °C until LC-MS analysis. Fully deuterated samples were prepared by overnight incubation of in 6 M deuterated guanidinium-hydrochloride (final deuterium content of 90 atom%).

The quenched deuterated proteins (30 pmol) were loaded onto a refrigerated HDX-UPLC system coupled to a hybrid Q-TOF Synapt G2 mass spectrometer (Waters, Milford, USA). The UPLC system was operated 0 °C and equipped with an in-house packed pepsin column with a 60 µl internal volume (IDEX, Oak Harbor, USA) containing pepsin immobilized on agarose (Thermo Scientific Pierce, Rockford, USA), a trap C18 column (ACQUITY UPLC BEH C18 1.7 µm VanGuard column (Waters, Milford, USA) and an analytical C18 column (ACQUITY UPLC BEH C18 1.7 µm, 1x100mm column (Waters, Milford, USA)). Proteins were digested in-line at a temperature of 20 °C and desalted on the trap column with a flow rate of 200 µl mobile phase A (0.23% FA). Peptic peptides were eluted from the trap column and across the analytical column at a flow of 40 µl/min and a 7 min gradient from 8 % to 40 % mobile phase B (ACN, 0.23% FA) and into the mass spectrometer for mass analysis. The ESI source was operated in positive ion mode the instrument was enabled for ion mobility analysis. A reference lock-spray signal of Glu-Fibrinopeptide (Sigma-Aldrich, St. Louis, USA) was acquired for internal calibration. Identification of peptides was done by a combination of MSe, HDMSe and DDA MS/MS. Peptide identifications were made through database searching in PLGS ver. 2.5 and HDX-MS data was processed in DynamX ver. 2.2.1. HDX-MS data of overlapping peptides were only used to localize deuterium uptake to smaller segments if the back-exchange of the fully deuterated antibody peptides was similar (below 7%) (Sheff, J., et al., J. Am. Soc. Mass Spectrom. 24 (2013) 1006-1015).

### Example 10

### Hydrogen/Deuterium exchange mass spectrometry with ETD

Deuterated samples were prepared by the same procedure as in the previous Example except the injection amount was adjusted to 100 pmol antibody and a five-fold dilution into D₂O buffer (to a final 80 atom% deuterium) was employed resulting in an antibody concentration of 4 pmol/µl and an FcRn concentration of 14 pmol/µl and 85 % bound antibody during labeling. HDX-ETD was performed in a targeted manner on selected antibody peptide fragments with differential deuterium uptake between the FcRn bound and unbound state. The ESI source and source T-wave was operated at settings optimized for minimal H/D scrambling as described in Rand, K.D., et al. (J. Am. Soc. Mass Spectrom. 22 (2011) 1784-1793) with the following parameters: capillary voltage 2.8 kV, desolvation gas flow 800 L/h, cone gas flow 0 L/h , source temperature 90 °C, desolvation gas temperature 300 °C, sampling cone 20 V, extraction cone 2 V, T-wave Trap wave velocity 300 m/s, wave height 0.2 V. ETD was performed in the trap T-wave using 1,4-dicyanobenzene (Sigma-Aldrich, St. Louis, USA) as the ETD reagent. 1,4-dicyanobenzene was introduced into the anion source using a nitrogen makeup flow of 20 ml/min over the reagent crystals stored in a sealed container. The radical anions were generated via glow discharge with a current of 40 µA and make up gas flow of 20 ml/min. ETD data was analyzed by determining the average masses of the c- and z-type ETD fragment ions. The deuterium content was calculated by subtracting the deuterium content of the unlabeled product ions from the deuterium content of the deuterated samples. The absence of H/D scrambling was verified by monitoring the loss of ammonia from the charged reduced species in ETD spectra recorded of peptic peptides from a fully deuterated antibody sample as described in Rand, K.D., et al. (Anal. Chem. 82 (2010) 9755-9762).

## Claims

1. A method for selecting a full length antibody comprising the following steps:
a) generating from a parent full length antibody a plurality of full length antibodies by randomizing one or more amino acid residues selected from the amino acid residues at positions 1-23 in the heavy chain variable domain - numbering according to Kabat - at positions 55-83 in the light chain variable domain - numbering according to Kabat - at positions 145-174 in the first heavy chain constant domain - numbering according to EU index - and at positions 180-197 in the first heavy chain constant domain - numbering according to EU index -,
b) determining the binding strength of each of the full length antibodies from the plurality of antibodies to the human neonatal Fc receptor (FcRn), and
c) selecting a full length antibody from the plurality of full length antibodies that has a different binding strength to the FcRn than the parent full length antibody.

## Patentansprüche

1. Verfahren zur Auswahl eines Volllängenantikörpers, umfassend die folgenden Schritte:
a) Erzeugen einer Vielzahl an Volllängenantikörpern durch Randomisieren eines oder mehrerer Aminosäurereste, ausgewählt aus den Aminosäureresten an den Positionen 1-23 in der variablen Domäne der schweren Kette, Nummerierung gemäß Kabat, an den Positionen 55-83 in der variablen Domäne der leichten Kette, Nummerierung gemäß Kabat, an den Positionen 145-174 in der ersten konstanten Domäne der schweren Kette, Nummerierung gemäß EU-Index, und an den Positionen 180-197 in der ersten konstanten Domäne der schweren Kette, Nummerierung gemäß EU-Index, ausgehend von einem Volllängen-Ausgangsantikörper,
b) Bestimmen der Bindungsstärke jedes der Volllängenantikörper aus der Vielzahl an Antikörpern an den humanen neonatalen Fc-Rezeptor (FcRn) und
c) Auswählen eines Volllängenantikörpers aus der Vielzahl an Volllängenantikörpern, der eine andere Bindungsstärke an den FcRn als der Volllängen-Ausgangsantikörper aufweist.

## Revendications

1. Procédé de sélection d'un anticorps pleine longueur comprenant les étapes suivantes :
a) la génération à partir d'un anticorps pleine longueur parent d'une pluralité d'anticorps pleine longueur en randomisant un ou plusieurs résidus d'acides aminés choisis parmi les résidus d'acides aminés aux positions 1 à 23 dans la numérotation de Kabat du domaine variable de la chaîne lourde, aux positions 55 à 83 dans la numérotation de Kabat du domaine variable de la chaîne légère, aux positions 145 à 174 dans la numérotation selon l'indice EU du premier domaine constant de la chaîne lourde et aux positions 180 à 197 dans la numérotation selon l'indice EU du premier domaine constant de la chaîne lourde,
b) la détermination de la force de liaison de chacun des anticorps pleine longueur parmi la pluralité d'anticorps au récepteur néonatal Fc humain (FcRn), et
c) la sélection d'un anticorps pleine longueur parmi la pluralité d'anticorps pleine longueur qui présente une force de liaison au FcRn différente de celle de l'anticorps pleine longueur parent.
